# EUROPÄISCHE PATENTSCHRIFT

(11) **EP 0 510 526 B1**
(45) Veröffentlichungstag und Bekanntmachung des Hinweises auf die Patenterteilung: **04.12.1996**
(21) Anmeldenummer: 92106602.3
(22) Anmeldetag: 16.04.1992
(51) Int. Cl.: C07D 239/46, C07D 239/42, C07D 405/12, A61K 31/505

(54) **Verwendung von Sulfonamiden als Heilmittel und neue Sulfonamide**
Use of sulfonamides as drug and new sulfonamides
Utilisation de sulfonamides comme médicament et nouvelles sulfonamides

(30) Priorität: 25.04.1991 CH 1242/91; 06.02.1992 CH 343/92
(43) Veröffentlichungstag der Anmeldung: 28.10.1992
(73) Patentinhaber: F. Hoffmann-La Roche AG, 4002 Basel (CH)
(72) Erfinder: Burri, Kaspar, Ch-4102 Binningen (CH); Clozel, Martine, F-68300 St. Louis (FR); Fischli, Walter, Ch-4123 Allschwil (CH); Hirth, Georges, F-68330 Huningue (FR); Löffler, Bernd Michael, W-7813 Oberrimsingen (DE); Ramuz, Henri, CH-4127 Birsfelden (CH)
(74) Vertreter: Grossner, Lutz, Dr.

(56) Entgegenhaltungen:
- DE-A- 1 545 944
- CHEMICAL ABSTRACTS, vol. 88, 1978, Columbus, Ohio, US; abstract no. 121105P, L. A. GRYGORIAN ET AL.: 'DERIVATIVES OF ARENESULFONIC ACIDS.IX.SYNTHESIS OF 4- SULFAMIDO-5-(P-ALKOXYBENZYL)PYRIMIDINES.' Seite 540 ;Spalte 2 ;

## Beschreibung

Die vorliegende Erfindung betrifft die Verwendung von Sulfonamiden der Formel 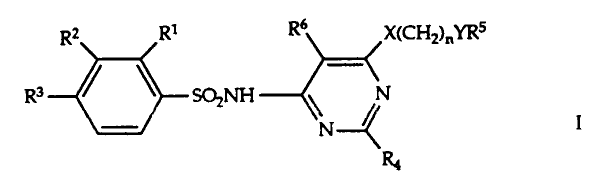 worin
- R¹: Wasserstoff, nieder-Alkyl, nieder-Alkoxy, nieder-Alkylthio, Halogen oder Trifluormethyl;
- R²: Wasserstoff, Halogen, nieder-Alkoxy, Hydroxy-nieder-alkoxy oder Trifluormethyl;
- R³: Wasserstoff, Hydroxy, Halogen, Alkylthio, Cycloalkyl, Hydroxynieder-alkyl, Hydroxy-nieder-alkoxy, Hydroximino-nieder-alkyl, nieder-Alkenyl, Oxo-nieder-alkyl, Trifluormethyl, Trifluormethoxy, nieder-Alkoxy, nieder-Alkoxy-nieder-alkoxy oder Aryl-nieder-alkoxy;
- R² und R³: zusammen Butadienyl;
- R⁴: Wasserstoff, nieder-Alkyl, Aryl oder Heteroaryl;
- R⁵: Wasserstoff, nieder-Alkanoyl, Benzoyl, Heterocyclyl-carbonyl oder Tetrahydropyran-2-yl;
- R⁶: einen Rest der Formel 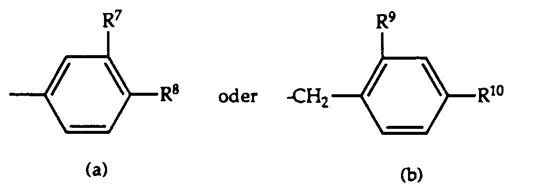
- R⁷: Wasserstoff, nieder-Alkoxy oder Nitro;
- R⁸: Wasserstoff, Halogen, nieder-Alkyl, nieder-Alkoxy, nieder-Alkylthio, Nitro, Hydroxy, Amino oder Trifluormethyl;
- R⁷ und R⁸: zusammen Butadienyl;
- R⁹: Wasserstoff, Halogen, nieder-Alkyl, nieder-Alkoxy, nieder-Alkylthio, oder Trifluormethyl;
- R¹⁰: Wasserstoff, Halogen, nieder-Alkyl, nieder-Alkoxy oder nieder-Alkylthio;
- X und Y: unabhängig voneinander O, S oder NH; und
- n: 2, 3 oder 4 bedeuten;
und Salzen davon als Wirkstoffe bei der Herstellung von Heilmitteln zur Behandlung von Kreislauferkrankungen, insbesondere Hypertonie, Ischämie, Vasospasmen und Angina pectoris.

Der hier verwendete Ausdruck "nieder" bezeichnet Gruppen mit 1-7 C-Atomen, vorzugsweise 1-4 C-Atomen. Alkyl-, Alkoxy-, Alkylthio- und Alkenylgruppen sowie Alkylgruppen als Bestandteile von Alkanoylgruppen können geradkettig oder verzweigt sein. Beispiele solcher Alkylgruppen sind Methyl, Aethyl, Propyl, Isopropyl, Butyl, sek. und tert.Butyl. Beispiele von Alkenylgruppen sind Vinyl und Allyl. Aryl-nieder-alkoxy ist beispielsweise Benzyloxy. Halogen bezeichnet Fluor, Chlor, Brom und Jod, wobei Chlor bevorzugt ist. Beispiele von Arylresten sind Phenyl- und substituierte Phenylreste, wobei als Substituenten insbesondere Halogen, Alkyl und Alkoxy in Betracht kommen. Beispiele von Heteroarylresten sind insbesondere monocyclische 5- und 6-gliedrige heteroaromatische Reste mit Stickstoff oder Schwefel als Heteroatom, wie Pyrimidinyl, Pyridyl, Pyrazinyl, Pyridazinyl und Thienyl. Heterocyclyl-carbonylreste sind z.B. 2-, 3- oder 4-Pyridylcarbonyl, 3-Methylisoxazol-5-ylcarbonyl, 2- oder 3-Furoyl, und 2- oder 3-Thenoyl.

Aus der Patentpublikation DE 1 545 944 sind Sulfonamide bekannt, die unter die oben angegebene Formel I fallen. Diese bekannten Sulfonamide wirken blutzuckersenkend. Ueberraschend wurde nun gefunden, dass die Verbindungen der oben angegebenen Formel I Hemmstoffe für Endothelin-Rezeptoren sind. Die Verbindungen der Formel I können daher zur Behandlung von Erkrankungen, die mit Endothelin-Aktivitäten assoziiert sind, insbesondere Kreislauferkrankungen, wie Hypertonie, Ischämie, Vasospasmen und Angina pectoris verwendet werden.

Eine bevorzugte Gruppe von Verbindungen innerhalb der Formel I sind diejenigen, worin R⁶ einen Rest der Formel 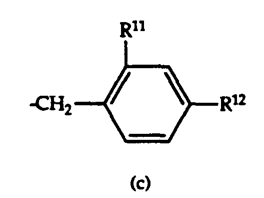 darstellt, und R¹¹ Halogen, nieder-Alkoxy, nieder-Alkylthio oder Trifluormethyl; und R¹² Wasserstoff oder nieder-Alkoxy bedeuten und R¹-R⁵, X, Y und n die oben angegebene Bedeutung haben.

Eine weitere bevorzugte Gruppe von Verbindungen innerhalb der Formel I sind diejenigen, worin R⁶ einen Rest der Formel 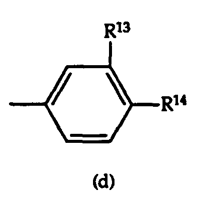 darstellt, und R¹³ Wasserstoff, nieder-Alkoxy oder Nitro; und R¹⁴ Wasserstoff, Halogen, nieder-Alkyl; nieder-Alkoxy, nieder-Alkylthio oder Nitro bedeuten, oder R¹³ und R¹⁴ gemeinsam Butadienyl bedeuten, und R¹-R⁵, X, Y und n die oben angegebene Bedeutung haben.

Die Verbindungen der Formel I können dadurch hergestellt werden, dass man eine Verbindung der Formel 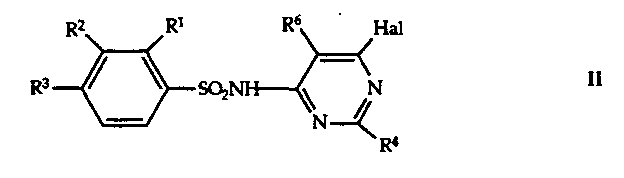 worin R¹, R², R³, R⁴ und R⁶ die oben genannte Bedeutung haben und Hal Halogen ist,
mit einer Verbindung der Formel

MX(CH₂)ₙYR⁵ III

worin X, Y, n und R⁵ die oben genannte Bedeutung haben, und M ein Alkalimetall darstellt,
umsetzt, und gegebenenfalls in der erhaltenen Verbindung der Formel I anwesende Substituenten abwandelt und/oder die erhaltene Verbindung der Formel I in ein Salz überführt.

In einer bevorzugten Ausführungsform des Verfahrens geht man von Verbindungen der Formeln II oder V aus, in denen R⁶ einen der oben defininierten Reste (c) oder (d) darstellt.

Die Umsetzung einer Verbindung der Formel II mit einer Verbindung der Formel III wird zweckmässig unter Verwendung des der Verbindung III zugrundeliegenden Glykols durchgeführt, also z.B. in Aethylenglykol, wenn n = 2 ist. Das Alkalimetall M ist vorzugsweise Natrium. Die Reaktion wird zweckmässig unter Erwärmen, z.B. auf 70-120°C, vorgenommen. In einer bevorzugten Ausführungsform setzt man als Verbindung der Formel III das Mono-Natriumsalz von Aethylen-, Propylen- oder Butylenglykol ein.

In der so erhaltenen Verbindung der Formel I können darin anwesende Substituenten abgewandelt werden. Beispielsweise kann eine Hydroxygruppe R⁵ verestert oder veräthert werden. Eine Nitrogruppe kann zur Aminogruppe reduziert werden. Eine nieder-Alkenylgruppe R³ kann z.B. mittels OsO₄ oder NaJO₄ zur Carbonylgruppe bzw. zu einer Alkanongruppe oxidiert werden; die so gebildete Carbonylgruppe kann, z.B. mittels Natriumborhydrid, zur Hydroxygruppe reduziert oder mit einer Alkyl-Grignard-Verbindung zu einem entsprechenden tertiären Alkohol umgesetzt oder mit Hydroxylamin in das Oxim übergeführt werden. Diese Umsetzungen können in an sich bekannter Weise vorgenommen werden, wobei eine Hydroxygruppe R⁵ zuvor in eine Aethergruppe, z.B. den Tetrahydropyranyläther, oder eine Estergruppe, z.B. das Azetat, umgewandelt wird. Diese Gruppen können gewünschtenfalls wieder in an sich bekannter Weise abgespalten werden; andererseits kann eine Abwandlung einer Hydroxygruppe R⁵ durch Veresterung oder Verätherung auch ohne nachfolgende Abwandlung anderer reaktiver Gruppen im Molekül vorgenommen werden. Die Verbindungen der Formel I können in an sich bekannter Weise in Salze, z.B. Alkalisalze wie Na- und K-Salze überführt werden.

Die als Ausgangsmaterial eingesetzten Verbindungen der Formeln II und III können, soweit sie nicht bekannt sind oder ihre Herstellung nachstehend beschrieben ist, in Analogie zu bekannten bzw. den nachstehend beschriebenen Methoden hergestellt werden.

Die Verbindungen der Formel II können gemäss dem nachstehenden Reaktionsschema hergestellt werden:

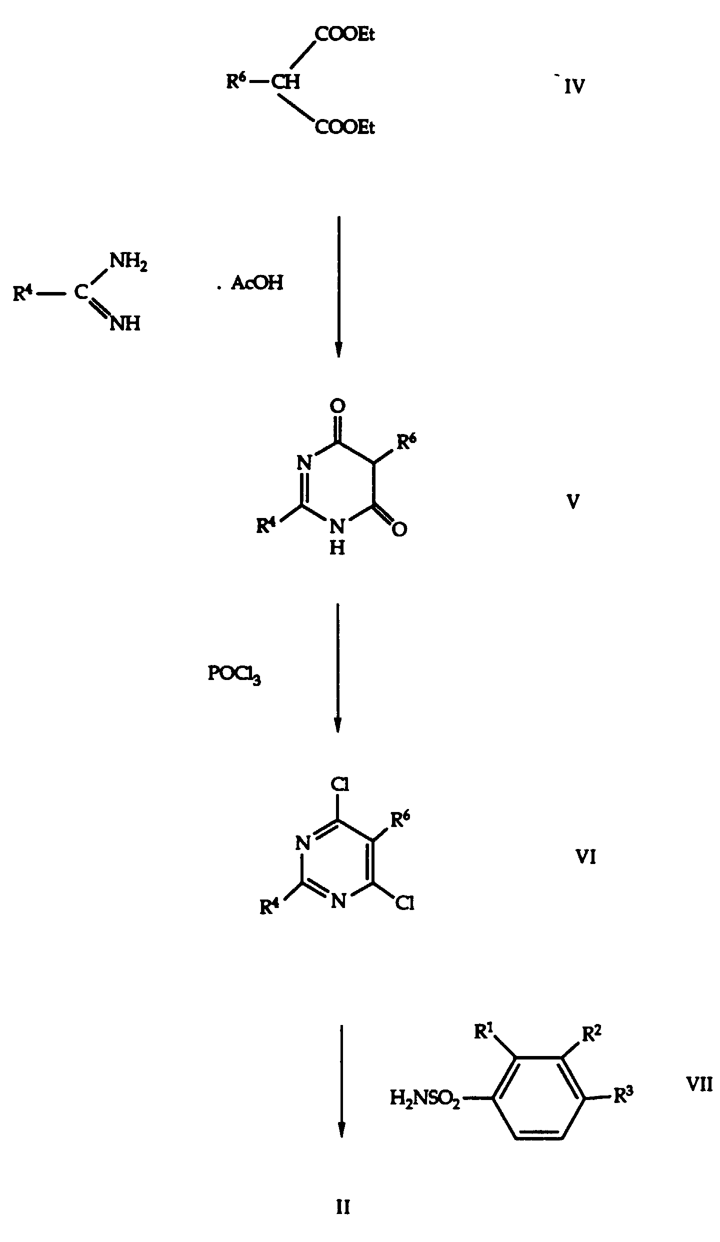

Kondensation der Verbindung IV mit Formamidinacetat oder einer homologen Verbindung wie Acetamidinacetat oder -hydrochlorid liefert das Pyrimidindion V. Mit Phosphoroxichlorid erhält man daraus die Dichlorverbindung VI, die bei der Umsetzung mit der Verbindung VII die Verbindung II liefert. Alle diese Umsetzungen sind Standardoperationen und können unter für solche Reaktionen üblichen und dem Fachmann geläufigen Bedingungen ausgeführt werden. Die Verbindungen IV, in denen R⁶ einen Rest (a) darstellt, können aus entsprechenden Phenylessigsäureestern der Formel R⁶CH₂COOEt durch Umsetzung mit Diäthylcarbenat in Gegenwart von Natriumäthylat erhalten werden. Die Verbindungen IV, in denen R⁶ einen Rest (b) darstellt, können durch Knoevenagel-Kondensation von Malonsäurediäthylester mit einem entsprechenden Aldehyd R⁶CHO und nachfolgende Hydrierung des Kondensationsprodukts hergestellt werden.

Die Hemmwirkung der Verbindungen der Formel I auf Endothelinrezeptoren kann mit der nachstehend beschriebenen Versuchsanordnungen gezeigt werden:

### I: Hemmung der Endothelin-Bindung an Human-Placentamembranen (vgl. Life Sci 44:1429 (1989))

Humanplacenta wird in 5 mM Tris-Puffer, pH 7.4, der 1 mM MgCl₂ und 250 mM Sucrose enthält, homogenisiert. Das Homogenisat wird mit 3000 g 15 Minuten bei 4°C zentrifugiert, der die Plasmamembranfraktion enthaltende Ueberstand mit 72000 g 30 Minuten zentrifugiert und der Bodenkörper mit 75 mM Tris-Puffer, pH 7.4, der 25 mM MgCl₂ enthält, gewaschen. Danach wird der aus jeweils 10 g Originalgewebe erhaltene Bodenkörper in 1 ml 75 mM Tris-Puffer pH 7.4, enthaltend 25 mM MgCl₂ und 250 mM Sucrose, suspendiert und in 1-ml-Aliquots bei -20°C eingefroren.

Für den Bindungs-Assay werden die eingefrorenen Membranpräparate aufgetaut und nach 10 Minuten Zentrifugieren mit 25000 g bei 20°C in Assay-Puffer (50 mM Tris-Puffer, pH 7.4, enthaltend 25 mM MnCl₂, 1 mM EDTA und 0,5% Rinderserumalbumin) resuspendiert. 100 µl dieser Membransuspension, enthaltend 70 µg Protein, werden mit 50 µl ¹²⁵I-Endothelin (spezif. Aktivität 2200 Ci/mMol) in Assay-Puffer (25000 cpm, Endkonzentration 20 pM) und 100 µl Assay-Puffer, der variierende Konzentrationen der Testverbindung enthält, inkubiert. Die Inkubation wird 2 Stunden bei 20°C oder 24 Stunden bei 4°C durchgeführt. Die Trennung von freiem und Membran-gebundenen Radioliganden wird durch Filtration über Glasfaserfilter vorgenommen.

In der Tabelle 1 ist die in dieser Versuchsanordnung ermittelte Hemmwirkung von Verbindungen der Formel I als IC₅₀ angegeben, d.h., als Konzentration [µM] die erforderlich ist, 50% der spezifischen Bindung von ¹²⁵I-Endothelin zu hemmen.

**Tabelle 1**

| Verbindung von Beispiel | IC₅₀[µM] |
|---|---|
| 1 | 5 |
| 54 | 3 |
| 63 | 1,6 |
| 64 | 2 |
| 66 | 0,5 |
| 83 | 0,7 |
| 84 | 1 |

### II. Hemmung Endothelin-induzierter Kontraktionen an isolierten Aortenringen der Ratte

Aus der Thoraxaorta von erwachsenen Wistar-Kyoto-Ratten wurden Ringe von 5 mm Länge herausgeschnitten. Das Endothel wurde durch leichtes Reiben der Innenfläche entfernt. Jeder Ring wurde bei 37°C in 10 ml Krebs-Henseleit-Lösung unter Begasung mit 95% O₂ und 5% CO₂ in ein isoliertes Bad eingetaucht. Die isometrische Spannung der Ringe wurde gemessen. Die Ringe wurden auf eine Vorspannung von 3 g gedehnt. Nach 10 Minuten Inkubation mit der Testverbindung oder Vehikel wurden kumulative Dosen von Endothelin-1 zugegeben. Die Aktivität der Testverbindung wurde durch Berechnung des Dosisverhältnisses, d.h. der durch 100 µM Testverbindung induzierten Rechtsverschiebung (Verschiebung zu höheren Werten) der EC₅₀ von Endothelin bestimmt, wobei EC₅₀ die für eine halbmaximale Kontraktion erforderliche Endothelin-Konzentration bezeichnet. Je grösser dieses Dosisverhältnis ist, desto potenter hemmt die Testverbindung die biologische Wirkung von Endothelin-1. Die EC₅₀ von Endothelin in Abweserheit von Testverbindungen ist 0,3 nM.

Die mit Verbindungen der Formel I so erhaltenen Werte für die Rechtsverschiebung der EC₅₀ von Endothelin sind in Tabelle 2 angegeben.

**Tabelle 2**

| Verbindung von Beispiel | Dosisverhältnis (Rechtsverschiebung) |
|---|---|
| 1 | 30 |
| 54 | 21 |
| 63 | 23 |
| 64 | 19 |
| 66 | 96 |
| 83 | 86 |
| 84 | 106 |

### III. Die Hemmwirkung der Verbindungen der Formel I auf die Vasokonstriktion kann in vivo an der Ratte in der nachstehend beschriebenen Versuchsanordnung beobachtet werden:

Ratten wurden mit Na-Thiobutabarbital (100 mg/kg i.p.) anästhesiert. Ueber die Femoralarterie wurde ein Katheter zur Messung des systemischen arteriellen Blutdrucks und über die Femoralvene ein Katheter in die Vena cava inferior zur Injektion der Testverbindungen gelegt. Eine Dopplersonde wurde um die linke Nierenarterie gelegt und mit einem Doppler-Messgerät verbunden. Durch 45 Minuten langes Abklemmen der linken Nierenarterie am Austrittsort wurde eine renale Ischämie erzeugt. Die Testverbindungen wurden 10 Minuten vor der Einleitung der Ischämie intraarteriell (i.a.) in Dosen von 5 mg/kg oder intravenös (i.v.) in Dosen von 10 mg/kg verabreicht. In Kontrollversuchen war die renale Durchblutung im Vergleich zum präischämischen Wert um 43±4% vermindert.

Die mit zwei Verbindungen der Formel I erhaltenen Messwerte sind in Tabelle 3 angegeben.

**Tabelle 3**

| Verbindung von Beispiel | % Abnahme der renalen Durchblutung |
|---|---|
| 1 i.a. | 7 |
| 83 i.v. | 29 |

Die Verbindungen der Formel I können aufgrund ihrer Fähigkeit, die Endothelinbindung zu hemmen, als Mittel zur Behandlung von Erkrankungen verwendet werden, die mit die Vasokonstriktion erhöhenden Vorgängen assoziiert sind. Beispiele solcher Erkrankungen sind Bluthochdruck, Koronarerkrankungen, Herzinsufftzienz, renale und myocardiale Ischämie, Niereninsuffizienz, Dialyse, cerebrale Ischämie, Hirninfarkt, Migräne, subarachnoidale Hämorrhagie, Raynaud-Syndrom und pulmonärer Hochdruck. Sie können ebenfalls bei Atherosklerose, Verhinderung der Restenosierung nach Ballon-induzierter Gefässdilatation, Entzündungen, Magen- und Duodenalulcera, Ulcus cruris, Gram-negativer Sepsis, Schock, Glomerulonephritis, Nierenkolik, Glaukom, Asthma, bei der Therapie und Prophylaxe diabetischer Komplikationen und Komplikationen bei der Verabreichung von Cyclosporin, sowie anderen, mit Endothelin-Aktivitäten assoziierten Erkrankungen Anwendung finden.

Die Verbindungen der Formel I können oral, rectal, parenteral, z.B. intravenös, intramuskulär, subcutan, intrathecal oder transdermal; oder sublingual oder als ophthalmologische Zubereitung, oder als Aerosol verabreicht werden. Beispiele von Applikationsformen sind Kapseln, Tabletten, oral verabreichbare Suspensionen oder Lösungen, Suppositorien, Injektionslösungen, Augentropfen, Salben oder Spraylösungen.

Eine bevorzugte Anwendungsform ist die intravenöse, intramuskuläre oder orale Applikation. Die Dosierung, in denen die Verbindungen der Formel I in wirksamen Mengen verabreicht werden, hängen von der Art des spezifischen Wirkstoffs, dem Alter und den Bedürfnissen des Patienten und der Applikationsweise ab. Im allgemeinen kommen Dosierungen von etwa 0,1-100 mg/kg Körpergewicht pro Tag in Betracht. Die die Verbindungen der Formel I enthaltenden Präparate können inerte oder auch pharmakodnymisch aktive Zusätze enthalten. Tabletten oder Granulate z.B. können eine Reihe von Bindemitteln, Füllstoffen, Trägersubstanzen oder Verdünnungsmitteln enthalten. Flüssige Präparate können beispielsweise in Form einer sterilen, mit Wasser mischbaren Lösung vorliegen. Kapseln können neben dem Wirkstoff zusätzlich ein Füllmaterial oder Verdickungsmittel enthalten. Des weiteren können geschmacksverbessernde Zusätze, sowie die üblicherweise als Konservierungs-, Stabilisierungs-, Feuchthalte- und Emulgiermittel verwendeten Stoffe, ferner auch Salze zur Veränderung des osmotischen Druckes, Puffer und andere Zusätze vorhanden sein.

Die vorstehend erwähnten Trägersubstanzen und Verdünnungsmittel können aus organischen oder anorganischen Stoffen, z.B. aus Wasser, Gelatine, Milchzucker, Stärke, Magnesiumstearat, Talkum, Gummi arabicum, Polyalkylenglykolen und dergleichen bestehen. Voraussetzung ist, dass alle bei der Herstellung der Präparate verwendeten Hilfsstoffe untoxisch sind.

Die nachstehenden Beispiele 1-104 beschreiben die Herstellung von erfindungsgemäss verwendbaren Verbindungen der Formel I

### Beispiel 1

Eine Lösung von 0,046 g Na in 1,5 ml abs. Aethylenglykol wurde mit 0,216 g N-[6-Chlor-5-(p-chlorphenyl)-4-pyrimidinyl]-α,α,α-trifluor-p-toluolsulfonamid unter Feuchtigkeitsausschluss versetzt und 3 Stunden auf 100°C erwärmt, danach auf Raumtemperatur abgekühlt und mit 2,3 ml 1N HCl versetzt. Das Gemisch wurde in Aethylacetat aufgenommen, die organischen Extrakte mit Wasser gewaschen, getrocknet und unter vermindertem Druck eingedampft. Der zurückbleibende Niederschlag wurde aus CH₂Cl₂, Isopropyläther und n-Hexan umkristallisiert und lieferte N-[5-(p-Chlorphenyl)-6-(2-hydroxyäthoxy)-4-pyrimidinyl]-α,α,α-trifluor-p-toluolsulfonamid, Schmelzpunkt 160-162°C.

Das Ausgangsmaterial wurde wie folgt hergestellt

Eine Lösung von 1,052 g α,α,α-Trifluorbenzolsulfonamid-Kalium und 0,520 g 4,6-Dichlor-5-(p-chlorphenyl)pyrimidin (Chem. Abstr. 63, 18078-HO₄) in 6 ml abs. DMF wurde 4 Stunden auf 100°C erwärmt, danach auf Raumtemperatur abgekühlt und mit 5 ml 1N HCl versetzt. Das Gemisch wurde in Aethylacetat aufgenommen, die organischen Extrakte mit Wasser gewaschen, getrocknet und unter vermindertem Druck eingedampft. Man erhielt N-[6-Chlor-5-(p-chlorphenyl)-4-pyrimidinyl]-α,α,α-trifluor-p-toluolsulfonamid als weisse Substanz vom Schmelzpunkt 275°C (aus Acetonitril).

### Beispiel 2

In Analogie zu Beispiel 1 wurde aus N-[6-Chlor-5-(p-chlorphenyl)-4-pyrimidinyl]-p-(trifluormethoxy)benzolsulfonamid und Aethylenglykol-Na das N-[5-(p-Chlorphenyl)-6-(2-hydroxyäthoxy)-4-pyrimidinyl]-p-(trifluormethoxy)benzolsulfonamid, Schmelzpunkt 152°C (aus Isopropyläther) erhalten.

Das Ausgangsmaterial wurde aus 4,6-Dichlor-5-(p-chlorphenyl)pyrimidin und p-(Trifluormethoxy)benzolsulfonamid-Kalium, Schmelzpunkt 240-242°C erhalten.

### Beispiel 3

In Analogie zu Beispiel 1 wurde aus p-Chlor-N-[6-chlor-5-(m-chlorphenyl)-4-pyrimidinyl]benzolsulfonamid und Aethylenglykol-Na das p-Chlor-N-[5-(m-chlorphenyl)-6-(2-hydroxyäthoxy)-4-pyrimidinyl]benzolsulfonamid, Schmelzpunkt 178-180°C (aus Aceton-Isopropyläther) erhalten.

Das Ausgangsmaterial wurde wie folgt hergestellt:
a) Zu einer Lösung von 3,96 g Na und 100 ml abs. Methanol wurden 5,97 g Formamidinacetat gegeben. Nach dem Abkühlen der Lösung auf 10°C wurden nach und nach 15,51 g Diäthyl-(m-chlorphenyl)malonat gegeben. Nach 2,5 Stunden wurde das Lösungsmittel unter vermindertem Druck eingedampft, der Rückstand in Wasser gelöst und die Lösung mit Eisessig auf pH 5,0 eingestellt. Der resultierende Niederschlag wurde abgenutscht, mit Wasser, Aethanol und Aether gewaschen und unter vermindertem Druck bei 70°C getrocknet. Man erhielt 5-(m-Chlorphenyl)-4,6(1H,5H)-pyrimidindion, das direkt in die nächste Stufe eingesetzt wurde.
b) Eine Mischung aus 10,6 g 5-(m-Chlorphenyl)-4,6(1H,5H)-pyrimidindion, 36 ml POCl₃ und 5,8 ml N,N-Dimethylanilin wurde 3 Stunden am Rückfluss gekocht. Nach dem Eindampfen des Lösungsmittels unter vermindertem Druck wurde der Rückstand mit Eis versetzt und die Mischung mit Aether extrahiert. Die organischen Lösungsmittel wurden getrocknet und unter vermindertem Druck eingedampft. Der ölige Rückstand wurde in n-Hexan aufgenommen, wobei das 4,8-Dichlor-5-(m-chlorphenyl)-pyrimidin auskristallisierte. Schmelzpunkt 93-94°C.
c) Aus 4,6-Dichlor-5-(m-chlorphenyl)pyrimidin und p-Chlorbenzolsulfonamid-K wurde das p-Chlor-N-[6-Chlor-5-(m-chlorphenyl)-4-pydmidinyl]benzolsulfonamid, Schmelzpunkt 226-228°C (aus CH₃CN) erhalten.

### Beispiel 4

In Analogie zu Beispiel 1 wurde aus p-Chlor-N-[6-chlor-5-(p-fluorphenyl)-4-pyrimidinyl]benzolsulfonamid das p-Chlor-N-[5-(p-fluorphenyl)-6-(2-hydroxyäthoxy)-4-pyrimidinyl]benzolsulfonamid, Schmelzpunkt 208-212°C (aus CH₃CN) erhalten.

Das Ausgangsmaterial wurde wie folgt hergestellt:
a) In Analogie zu Beispiel 3, Abschnitt a), wurde aus Diäthyl-(p-fluorphenyl)malonat und Formamidinacetat das 5-(p-Fluorphenyl)-4,6(1H,5H)-pyrimidindion als Feststoff erhalten, der direkt in die nächste Stufe eingesetzt wurde.
b) In Analogie zu Beispiel 3, Abschnitt b) wurde aus 5-(p-Fluorphenyl)-4,6(1H,5H)-pyrimidindion und POCl₃ das 4,6-Dichlor-5-(p-fluorphenyl)pyrimidin, Schmelzpunkt 98-99°C (aus n-Hexan) erhalten.
c) In Analogie zu Beispiel 3, Abschnitt c) wurde aus 4,6-Dichlor-5-(p-fluorphenyl)pyrimidin und p-Chlorphenylsulfonamid-K das p-Chlor-N-[6-chlor-5-(p-fluorphenyl)-4-pyrimidinyl]benzolsulfonamid, Schmelzpunkt 251-254°C (aus Methylenchlorid-Isopropyläther) erhalten.

### Beispiel 5

In Analogie zu Beispiel 1 wurde aus N-(6-Chlor-5-(p-cMorphenyl)-4-pyrimidinyl]-p-fluorbenzolsulfonamid und Aethylenglykol-Na das N-[5-(p-Chlorphenyl)-6-(2-hydroxyäthoxy)-4-pyrimidinyl]-p-fluorbenzolsulfonamid, Schmelzpunkt 181-183°C (aus Methylenchlorid-Isopropyläther) erhalten.

Das Ausgangsmaterial wurde aus 4,6-Dichlor-5-(p-chlorphenyl)pyrimidin und p-Fluorphenylsulfonamid hergestellt. Schmelzpunkt 244-246°C (aus Methylenchlorid-Isopropyläther).

### Beispiel 6

In Analogie zu Beispiel 1 wurde aus o-Chlor-N-[6-chlor-5-(p-chlorphenyl)-4-pyrimidinyl]benzolsulfonamid und Aethylenglykol-Na das o-ChlorN-[5-(p-chlorphenyl)-6-(2-hydroxyäthoxy)-4-pyrimidinyl]benzolsulfonamid, Schmelzpunkt 183-185°C (aus Aceton und Isopropyläther) erhalten.

Das Ausgangsmaterial wurde aus 4,6-Dichlor-5-(p-chlorphenyl)pyrimidin und o-Chlorphenylsulfonamid erhalten. Schmelzpunkt 230-234°C (aus CH₃CN).

### Beispiel 7

In Analogie zu Beispiel 1 wurde aus N-[6-Chlor-5-(p-äthylphenyl)-4-pyrimidinyl]-p-cydopentylbenzolsulfonamid und Aethylenglykol-Na das p-Cydopentyl-N-[6-(2-hydroxyäthoxy)-5-(p-äthylphenyl)-4-pyrimidinyl]benzolsulfonamid, Schmelzpunkt 145-146°C (aus Aceton und Isopropyläther) erhalten.

Das Ausgangsmaterial wurde aus 4,6-Dichlor-5-(p-äthylphenyl)pyrimidin und p-Cyclopentylbenzolsulfonamid hergestellt, Schmelzpunkt 178-180°C (aus Acetonitril und Isopropyläther).

### Beispiel 8

In Analogie zu Beispiel 1 wurde aus p-Chlor-N-[6-chlor-5-(3,4-dimethoxyphenyl)-4-pyrimidinyl]benzolsulfonamid und Aethylenglykol-Na das p-Chlor-N-[5-(3,4-dimethoxyphenyl)-6-(2-hydroxyäthoxy)-4-pyrinüdinyl]benzolsulfonamid, Schmelzpunkt 232-234°C (aus CH₃CN) erhalten.

Das Ausgangsmaterial wurde wie folgt hergestellt

In Analogie zu Beispiel 3, Abschnitt b), wurde aus 5-(3,4-Dimethoxyphenyl)-4,6(1H,5H)-pyrimidindion und POCl₃ das 4,6-Dichlor-5-(3,4-dimethoxyphenyl)pyrimidin, Schmelzpunkt 151-152°C (aus Cydohexan-Aether) hergestellt, aus dem mit p-Chlorphenylsulfonamid das p-Chlor-N-[6-chlor-5-(3,4-dimethoxyphenyl)-4-pyrimidinyl]benzolsulfonamid, Schmelzpunkt 201-203°C (aus CH₃CN) erhalten wurde.

### Beispiel 9

In Analogie zu Beispiel 1 wurde aus 3,4-Dichlor-N-[6-chlor-5-(p-chlorphenyl)pyrimidinyl]benzolsulfonamid und Aethylenglykol-Na das 3,4-Dichlor-N-[5-(p-chlorphenyl)-6-(2-hydroxyäthoxy)-4-pyrimidinyl]benzolsulfonamid, Schmelzpunkt 181°C (aus CH₃CN und Isopropyläther) erhalten.

### Beispiel 10

In Analogie zu Beispiel 1 wurde aus N-[6-Chlor-5-(p-chlorphenyl)-4-pyrimidinyl]-α,α,α-α',α',α'-hexafluormethyl-xylolsulfonamid das N-5-(p-Chlorphenyl)-6-(2-hydroxyäthoxy)-4-pyrimidinyl]-α,α,α-α',α',α'-hexafluor-3,5-xylolsulfonamid, Schmelzpunkt 156-158°C (aus Methylenchlorid/n-Hexan) erhalten.

Das Ausgangsmaterial wurde aus 4,6-Dichlor-5-(p-chlorphenyl)pyrimidin und 2,4-bis-Trifluormethylphenylsulfonamid hergestellt. Schmelzpunkt 132-135°C (aus Isopropyläther); Reinheit 92% (HPLC-Analyse).

### Beispiel 11

In Analogie zu Beispiel 1 wurde aus 3-Chlor-N-[6-chlor-5-(p-chlorphenyl)-4-pyrimidinyl]-4-fluorbenzolsulfonamid und einem Ueberschuss von Aethylenglykol-Na das 3-Chlor-N-[5-(p-chlorphenyl)-6-(2-hydroxyäthoxy)-4-pyrimidinyl]-4-(2-hydroxyäthoxy)benzolsulfonamid, Schmelzpunkt 138-140°C (aus Aceton-Isopropyläther) erhalten.

Das Ausgangsmaterial wurde aus 4,6-Dichlor-5-(p-chlorphenyl)pyrimidin und 3-Chlor-4-fluorphenylsulfonamid hergestellt. Schmelzpunkt 239°C (aus Methylenchlorid-Acetonitril).

### Beispiel 12

In Analogie zu Beispiel 1 wurde aus p-Chlor-N-(6-chlor-5-(p-nitrophenyl)-4-pyrimidinyl]benzolsulfonamid und Aethylenglykol-Na das p-Chlor-N-(6-(2-hydroxyäthoxy)-5-(p-nitrophenyl)-4-pyrimidinyl]benzolsulfonamid, Schmelzpunkt 223-225°C (aus Methylenchlorid-Isopropyläther) erhalten.

Das Ausgangsmaterial wurde aus 4-Chlor-5-(p-nitrophenyl)pyrimidin und p-Chlorphenylsulfonamid hergestellt; Schmelzpunkt 282-285°C (aus CH₃CN).

### Beispiel 13

In Analogie zu Beispiel 1 wurde aus p-Butoxy-N-[6-chlor-5-(p-chlorphenyl)-4-pyrimidinyl]benzolsulfonamid und Aethylenglykol-Na das p-Butoxy-N-[5-(p-chlorphenyl)-6-(2-hydroxyäthoxy)-4-pyrimidinyl]benzolsulfonamid, Schmelzpunkt >300°C (aus Isopropyläther), Reinheit 97,7% in HPLC-Analyse erhalten.

Das Ausgangsmaterial wurde aus 4,6-Dichlor-5-(p-chlorphenyl)pyrimidin und 4-n-Butoxyphenylsulfonamid hergestellt. Schmelzpunkt 234°C (aus CH₃CN).

### Beispiel 14

In Analogie zu Beispiel 1 wurde aus N-6-Chlor-[5-(p-chlorphenyl)-4-pyrimidinyl]-3,4-dimethoxybenzolsulfonamid und Aethylenglykol-Na das N-[5-(p-Chlorphenyl)-6-(2-hydroxyäthoxy)-4-pyrimidinyl]-3,4-dimethoxybenzolsulfonamid, Schmelzpunkt 130-132°C (aus Isopropyläther) erhalten.

Das Ausgangsmaterial wurde aus 4,6-Dichlor-5-(p-chlorphenyl)pyrimidin und 3,4-Dimethoxyphenylsulfonamid hergestellt. Schmelzpunkt 226°C (aus CH₃CN).

### Beispiel 15

In Analogie zu Beispiel 1 wurde aus o-Chlor-N-[6-chlor-5-(p-chlorphenyl)-4-pyrimidinyl]-α,α,α-trifluor-p-toluolsulfonamid und Aethylenglykol-Na das 2-Chlor-N-[5-(p-chlorphenyl)-6-(2-hydroxyäthoxy)-4-pyrimidinyl]-α,α,α-trifluor-p-toluolsulfonamid, Schmelzpunkt 131°C (aus Isopropyläther) erhalten.

Das Ausgangsmaterial wurde aus 4,6-Dichlor-5-(p-chlorphenyl)pyrimidin und 2-Chlor-α,α,α-trifluor-p-toluolsulfonamid hergestellt; Schmelzpunkt 234°C (aus Methylenchlorid-Acetonitril).

### Beispiel 16

In Analogie zu Beispiel 1 wurde aus 6-Chlor-N-[6-chlor-5-(p-chlorphenyl)-4-pyrimidinyl]-α,α,α-trifluor-m-toluolsulfonamid und Aethylenglykol-Na das 6-Chlor-N-[5-(p-chlorphenyl)-6-(2-hydroxyäthoxy)-4-pyrimidinyl]-α,α,α-trifluor-m-toluolsulfonamid, Schmelzpunkt 185-186°C (aus Isopropyläther) erhalten.

Das Ausgangsmaterial wurde aus 4,6-Dichlor-5-(p-chlorphenyl)pyrimidin und α,α,α-Trifluor-3-methyl-6-chlorphenylsulfonamid hergestellt; Schmelzpunkt 232°C (aus Isopropyläther).

### Beispiel 17

In Analogie zu Beispiel 1 wurde aus 2,3,4-Trichlor-N-[6-chlor-5-(p-chlorphenyl)-4-pyrimidinyl]benzolsulfonamid und Aethylenglykol-Na das 2,3,4-Trichlor-N-[(5-chlorphenyl)-6-(2-hydroxyäthoxy)-4-pyrimidinyl]benzolsulfonamid, Schmelzpunkt 209-211°C (aus Methylenchlorid-Isopropyläther) erhalten.

Das Ausgangsmaterial wurde aus 4,6-Dichlor-5-(p-chlorphenyl)pyrimidin und 2,3,4-Trichlorphenylsulfonamid hergestellt; Schmelzpunkt 278-280°C (aus CH₃CN).

### Beispiel 18

In Analogie zu Beispiel 1 wurde aus m-Chlor-N-[6-chlor-5-(p-chlorphenyl)-4-pyrimidinyl]benzolsulfonamid und Aethylenglykol-Na das m-Chlor-N-(p-chlorphenyl)-6-(2-hydroxyäthoxy)-4-pyrimidinyl]benzolsulfonamid, Schmelzpunkt 179-181°C (aus Acetonitril-Isopropyläther) erhalten.

Das Ausgangsmaterial wurde aus 4,6-Dichlor-5-(p-chlorphenyl)pyrimidin und 3-Chlorphenylsulfonamid hergestellt; Schmelzpunkt 219-221°C (aus CH₃CN).

### Beispiel 19

In Analogie zu Beispiel 1 wurde aus 2,4-Dichlor-N-[6-chlor-5-(p-chlorphenyl)-4-pyrimidinyl]benzolsulfonamid und Aethylenglykol-Na das 2,4-Dichlor-N-[5-(p-chlorphenyl)-6-(2-hydroxyäthoxy)-4-pyrimidinyl]benzolsulfonamid, Schmelzpunkt 165-167°C (aus CH₃CN) erhalten.

Das Ausgangsmaterial wurde aus 4,6-Dichlor-5-(p-chlorphenyl)pyrimidin und 2,4-Dichlorphenylsulfonamid hergestellt; Schmelzpunkt 252-254°C (aus CH₃CN).

### Beispiel 20

In Analogie zu Beispiel 1 wurde aus N-6-Chlor-5-(p-chlorphenyl)-α,α,α-trifluor-m-toluolsulfonamid und Aethylenglykol-Na das N-5-(p-Chlorphenyl)-6-(2-hydroxyäthoxy)-α,α,α-trifluor-m-toluolsulfonamid, Schmelzpunkt 148-150°C (aus Isopropyläther) erhalten.

Das Ausgangsmaterial wurde aus 4,6-Dichlor-5-(p-chlorphenyl)pyrimidin und α,α,α-Trifluor-m-toluolsulfonamid hergestellt; Schmelzpunkt 197-198°C.

### Beispiel 21

In Analogie zu Beispiel 1 wurde aus N-6-Chlor-5-(p-chlorphenyl)-α,α,α-trifluor-o-toluolsulfonamid und Aethylenglykol-Na das N-5-(p-Chlorphenyl)-6-(2-hydroxyäthoxy)-4-pyrimidinyl]-α,α,α-trifluor-o-toluolsulfonamid, Schmelzpunkt 182-184°C (aus CH₃CN-Isopropyläther) erhalten.

Das Ausgangsmaterial wurde aus 4,6-Dichlor-5-(p-chlorphenyl)pyrimidin und α,α,α-Trifluor-o-toluolsulfonamid hergestellt; Schmelzpunkt 191-193°C (aus CH₃CN).

### Beispiel 22

In Analogie zu Beispiel 1 wurde aus N-[6-Chlor-5-(p-äthylphenyl)-4-pyrimidinyl]-p-isopropylbenzolsulfonamid und Aethylenglykol-Na das N-[6-(2-Hydroxyäthoxy)-5-(p-äthylphenyl)-4-pyrimidinyl]-p-isopropyl]benzolsulfonamid, Schmelzpunkt 137-138°C (aus Acetonitril und Isopropyläther) erhalten.

Das Ausgangsmaterial wurde wie folgt hergestellt:

Aus Diäthyl-(p-äthylphenyl)malonat und Formamidinacetat wurde das 5-(p-Aethyl)-4,6(1H,5H)-pyrimidindion, Schmelzpunkt >270°C, und daraus mit POCl₃ das 4,6-Dichlor-5-(p-äthylphenyl)pyrimidin, Schmelzpunkt 48-49°C (aus n-Hexan) erhalten.

Umsetzung dieser Verbindung mit p-Isopropylbenzolsulfonamid lieferte das N-[6-Chlor-5-(p-äthylphenyl)-4-pyrimidinyl]-p-isopropylbenzolsulfonamid, Schmelzpunkt 187-188°C (aus Acetonitril und Isopropyläther).

### Beispiel 23

In Analogie zu Beispiel 1 wurde aus N-[6-Chlor-5-(p-chlorphenyl)-4-pyrimidinyl]-2-naphthalinsulfonamid und Aethylenglykol-Na das N-[5-(p-Chlorphenyl)-6-(2-hydroxyäthoxy)-4-pyrimidinyl]-2-naphthalinsulfonamid, Schmelzpunkt 137-138°C (aus CH₃CN und Isopropyläther) erhalten.

Das Ausgangsmaterial wurde aus 4,6-Dichlor-5-(p-chlorphenyl)pyrimidin und 2-Naphthalinsulfonamid hergestellt; Schmelzpunkt 265-269°C (aus CH₃CN).

### Beispiel 24

In Analogie zu Beispiel 1 wurde aus p-Chlor-N-[6-chlor-5-(m-nitrophenyl)-4-pyrimidinyl]benzolsulfonamid und Aethylenglykol-Na das p-Chlor-N-[6-(2-hydroxyäthoxy)-5-(m-nitrophenyl)-4-pyrimidinyl]benzolsulfonamid, Schmelzpunkt 186-187°C (aus CH₃CN und Isopropyläther) erhalten.

Das Ausgangsmaterial wurde aus 4,6-Dichlor-5-(m-nitrophenyl)pyrimidin und p-Chlorphenylsulfonamid hergestellt; Schmelzpunkt 261-263°C (aus CH₃CN).

### Beispiel 25

In Analogie zu Beispiel 1 wurde aus N-[6-Chlor-5-(m-nitrophenyl)-4-pyrimidinyl]-α,α,α-trifluor-p-toluolsulfonamid und Aethylenglykol-Na das α,α,α-Trifluor-N-[6-(2-hydroxyäthoxy)-5-(m-nitrophenyl)-4-pyrimidinyl]-p-toluolsulfonamid, Schmelzpunkt 194-195°C (aus Essigester/n-Hexan) erhalten.

Das Ausgangsmaterial wurde aus 4,6-Dichlor-5-(m-nitrophenyl)pyrimidin und α,α,α-Trifluor-p-toluolsulfonamid hergestellt; Schmelzpunkt 246-250°C (aus CH₃CN).

### Beispiel 26

In Analogie zu Beispiel 1 wurde aus p-(Benzyloxy)-N-[6-chlor-5-(p-chlorphenyl)-4-pyrimidinyl]benzolsulfonamid und Aethylenglykol-Na das p-(Benzyloxy)-N-[5-(p-chlorphenyl)-6-(2-hydroxyäthoxy)-4-pyrimidinyl]benzolsulfonamid, Schmelzpunkt 162-163°C (aus Aceton und Isopropyläther) erhalten.

Das Ausgangsmaterial wurde aus 4,6-Dichlor-5-(p-chlorphenyl)pyrimidin und p-(Benzyloxy)benzolsulfonamid hergestellt; Schmelzpunkt 233-236°C (aus Aceton und Essigester).

### Beispiel 27

Eine Lösung aus 512 mg p-(Benzyloxy)-N-[5-(p-chlorphenyl)-6-(2-hydroxyäthoxy)-4-pyrimidinylbenzolsulfonamid in 30 ml Eisessig wurde mit 2 ml 4N HCl in Dioxan und 100 mg 10% Palladium-Kohle versetzt. Die Mischung wurde unter Rühren hydriert, die Lösung danach abgenutscht, unter reduziertem Druck eingedampft und der Rückstand aus Isopropyläther und nochmals aus CH₃CN umkristallisiert. Man erhielt N-[5-(p-Chlorphenyl)-4-pyrimidinyl]-p-hydroxybenzolsulfonamid, Schmelzpunkt 231-232°C.

### Beispiel 28

In Analogie zu Beispiel 1 wurde aus N-[6-Chlor-5-(p-chlorphenyl)-4-pyrimidinyl]-p-(2-methoxyäthoxy)benzolsulfonamid und Aethylenglykol-Na das N-[5-(p-Chlorphenyl)-6-(2-hydroxyäthoxy)-4-pyrimidinyl]-p-(2-methoxyäthoxy)benzolsulfonamid, Schmelzpunkt 151-152°C (aus CH₃CN und Isopropyläther) erhalten.

Das Ausgangsmaterial wurde aus 4,6-Dichlor-5-(p-chlorphenyl)pyrimidin und p-(2-Methoxyäthoxy)benzolsulfonamid hergestellt; Schmelzpunkt 212-215°C (aus CH₃CN).

### Beispiel 29

In Analogie zu Beispiel 1 wurde aus N-[5-(p-Bromphenyl)-6-chlor-4-pyrimidinyl]-p-chlorbenzolsulfonamid und Aethylenglykol-Na das N-[5-(p-Bromphenyl)-6-(2-hydroxyäthoxy)-4-pyrimidinyl]-p-chlorbenzolsulfonamid, Schmelzpunkt 179-180°C (aus Aceton und Isopropyläther) erhalten.

Das Ausgangsmaterial wurde wie folgt hergestellt:

In Analogie zu Beispiel 3, Abschnitt a) wurde aus Diäthyl-p-bromphenylmalonat und Formamidinacetat, das 5-(p-Bromphenyl)-4,6(1H,5H)-pyrimidindion, Schmelzpunkt >270°C hergestellt. Nach dem Trocknen unter reduziertem Druck bei 80°C über Nacht wurde die Verbindung in die nächste Stufe eingesetzt.

In Analogie zu Beispiel 3, Abschnitt b) wurde aus 5-(p-Bromphenyl)-4,6(1H,5H)-pyrimidindion und POCl₃ das 5-(p-Bromphenyl)-4,6-dichlorpyrimidin, Schmelzpunkt 99-100°C (aus Hexan) hergestellt und daraus mit p-Chlorphenylsulfonamid das N-[5-(p-Bromphenyl)-6-chlor-4-pyrimidinyl]-p-chlorbenzolsulfonamid, Schmelzpunkt 266-268°C (aus CH₃CN).

### Beispiel 30

In Analogie zu Beispiel 1 wurde aus p-Chlor-N-(6-chlor-5-p-tolyl-4-pyrimidinyl)benzolsulfonamid und Aethylenglykol-Na das p-Chlor-N-[6-(2-hydroxyäthoxy)-5-p-tolyl-4-pyrimidinyl]benzolsulfonamid, Schmelzpunkt 162-165°C (aus Aceton und Isopropyläther) erhalten.

Das Ausgangsmaterial wurde wie folgt hergestellt:

In Analogie zu Beispiel 3, Abschnitt a) wurde aus Diäthyl-p-tolylmalonat und Formamidinacetat das 5-p-Tolyl-4,6(1H,5H)-pyrimidindion, Schmelzpunkt >270°C hergestellt. Nach dem Trocknen unter vermindertem Druck bei 80°C wurde die Substanz in die nächste Stufe eingesetzt.

In Analogie zu Beispiel 3, Abschnitt b) wurde aus 5-p-Tolyl-4,6(1H,5H)-pyrimidindion und POCl₃ das 4,6-Dichlor-5-p-tolylpyrimidin, Schmelzpunkt 81-82°C (aus Hexan) hergestellt und daraus mit p-Chlorphenylsulfonamid das p-Chlor-N-(6-chlor-5-p-tolyl-4-pyrimidinyl)benzolsulfonamid, Schmelzpunkt 229-230°C (aus Acetonitril).

### Beispiel 31

Eine Lösung von 237 mg N-[5-(p-Chlorphenyl)-6-(2-hydroxyäthoxy)-4-pyrimidinyl]-α,α,α-trifluor-p-toluolsulfonamid in 5 ml Methanol wurde mit 27,0 mg Natriummethylat und danach mit 5 ml Isopropyläther versetzt. Der weisse Niederschlag wurde abgenutscht und unter vermindertem Druck bei 50°C getrocknet. Man erhielt N-[5-(p-Chlorphenyl)-6-(2-hydroxyäthoxy)-4-pyrimidinyl]-α,α,α-trifluor-p-toluolsulfonamid-Natriumsalz als weissen Feststoff.

### Beispiel 32

Man gab 60 mg Natrium zu 2 ml Aethylenglykol bei 70°C. Danach wurden 223 mg N-[6-Chlor-5-(2,6-dimethoxybenzyl)-4-pyrimidinyl]-p-vinylbenzolsulfonamid zugesetzt und das Reaktionsgemisch 4,5 Stunden auf 150°C erhitzt. Das Aethylenglykol wurde unter vermindertem Druck abdestilliert, der Rückstand in EtOAc/H₂O aufgenommen und mit Aethylacetat einmal extrahiert. Danach wurde die wässrige Phase mit 1N HCl angesäuert und viermal mit Aethylacetat extrahiert. Die organische Phase wurde getrocknet, abfiltriert und unter vermindertem Druck eingeengt. Der Rückstand wurde über 50 g SiO₂ mit Methylenchlorid/Essigester 1:1 chromatographiert. Man erhielt 50 mg N-[5-(2,6-Dimethoxybenzyl)-6-(2-hydroxyäthoxy)-4-pyrimidinyl]-p-vinylbenzolsulfonamid, Schmelzpunkt 138-139°C.

Das Ausgangsmaterial wurde wie folgt hergestellt:
a) Ein Gemisch von 1,52 ml Malonsäurediäthylester, 1,66 g 2,6-Dimethoxybenzaldehyd, 0,1 ml Piperidin, 0,11 ml Eisessig und 100 ml Toluol wurde bei 110°C am Wasserabscheider 3,5 Stunden gekocht. Die Lösung wurde mit 10%iger NaHCO₃-Lösung extrahiert und mit gesättigter NaCl-Lösung nachgewaschen. Die organische Phase wurde getrocknet, abgenutscht und unter vermindertem Druck eingedampft. Man erhielt 2,8 g Diäthyl-(2,6-dimethoxybenzyliden)malonat als dunkelgelbes Oel.
b) Ein Gemisch von 2,8 g Diäthyl-(2,6-dimethoxybenzyliden)malonat, 0,6 g Palladium-Kohle, 50 ml Methanol und 50 ml Eisessig wurde über Nacht bei 25°C gerührt. Die Lösung wurde filtriert und eingeengt und der Rückstand in Aethylacetat aufgenommen und mit 20% NaHCO₃ und etwas Eis extrahiert. Danach wurde mit 1H HCl extrahiert, mit gesättigter NaCl-Lösung nachgewaschen, die organische Phase getrocknet und unter vermindertem Druck eingedampft. Das Rohprodukt wurde am Hochvakuum bei 170°C/0,6 mbar destilliert. Man erhielt 2,1 g Diäthyl-(2,6-dimethoxybenzyl)malonat.
c) Zu 0,14 g Natrium in 15 ml Aethanol wurden 0,23 g Formamidinacetat in 15 ml Aethanol gegeben. Das Reaktionsgemisch wurde 30 Minuten bei 25°C gerührt, und tropfenweise mit 0,62 g Diäthyl-(2,6-dimethoxybenzyl)malonat in 10 ml Aethanol versetzt. Nach 2 Tagen war kein Ausgangsmaterial mehr vorhanden. Der Rückstand wurde abgenutscht, in wenig Wasser gelöst und mit 1N HCl angesäuert. Die ausgefallenen Kristalle wurden abgenutscht und am Hochvakuum bei 90°C getrocknet. Man erhielt 0,175 g 5-(2,6-Dimethoxybenzyl)-4,6-pyrimidindiol vom Schmelzpunkt >245°C.
d) Ein Gemisch von 1,04 g 5-(2,6-Dimethoxybenzyl)-4,6-pyrimidindiol und 12 ml Phosphoroxychlorid wurde bei 85°C am Rückfluss 3 Stunden gekocht. Die Reaktionslösung wurde auf Eis gegossen und zweimal mit Methylenchlorid extrahiert. Die organische Phase wurde mit gesättigter NaCl-Lösung nachgewaschen, getrocknet, abfiltriert und unter vermindertem Druck eingeengt. Das Rohprodukt wurde aus Toluol/n-Hexan umkristallisiert. Man erhielt 0,41 g 4,6-Dichlor-5-(2,6-dimethoxybenzyl)pyrimidin, Schmelzpunkt 152-153°C.
e) Ein Gemisch von 80 mg 4,6-Dichlor-5-(2,6-dimethoxybenzyl)pyrimidin und 170 mg p-Vinylbenzolsulfonamid-mono-Kaliumsalz (J. Am. Chem. Soc. 1956, 78, 2169) aus dem entsprechenden Sulfonamid mit Kalium-t-butylat in abs. MeOH und 10 ml Dimethylformamid wurde 6 Stunden auf 100°C erhitzt. Danach liess man über Nacht auf 25°C abkühlen. Nun gab man 30 ml 0,5N HCl unter Rühren zur Reaktionslösung. Die ausgefallene Substanz wurde abgenutscht und aus Toluol/n-Hexan umkristallisiert. Man erhielt 25 mg N-(6-Chlor-5-(2,6-dimethoxybenzyl)-4-pydmidinyl)-p-vinylbenzolsulfonamid, Schmelzpunkt 197-198°C.

### Beispiel 33

Zu 10 ml Aethylenglykol (frisch über Na destilliert) wurden unter Feuchtigkeitsausschluss portionsweise 29 mg Natrium gegeben. Danach wurden 123 mg N-[6-Chlor-6-[o-(trifluormethyl)benzyl]-4-pyrimidinyl]-α,α,α-trifluor-p-toluolsulfonamid zugesetzt und das Reaktionsgemisch 3 Stunden auf 150°C erhitzt. Danach wurde das überschüssige Aethylenglykol unter vermindertem Druck abgedampft; der Rückstand in Wasser gelöst und mit Aethylacetat gewaschen. Die wässrige Phase wurde mit 1N Salzsäure auf pH 3,0 gestellt und mit Aethylacetat extrahiert. Die organische Phase wurde mit Wasser und gesättigter Kochsalzlösung gewaschen, getrocknet und unter vermindertem Druck eingedampft. Der Rückstand wurde über 30 g SiO₂ mit Methylenchlorid/Aethylacetat (1:1) chromatographiert. Man erhielt α,α,α-Trifluor-N-[6-(2-hydroxyäthoxy)-6-[o-(trifluormethyl)benzyl]pyrimidinyl]-p-toluolsulfonamid als weissen Schaum. MS: 521 (M); 456 (M-SO₂+H).

Das Ausgangsmaterial wurde wie folgt hergestellt:
a) Zu einer Lösung aus 14 g o-Trifluormethylbenzylalkohol in 80 ml abs. Toluol tropfte man bei 20-30°C eine Lösung aus 30 ml Phosphortribromid in 60 ml abs. Toluol. Anschliessend wurde das Reaktionsgemisch 2 Stunden bei Zimmertemperatur gerührt, das Toluol unter vermindertem Druck abdestilliert, der Rückstand in Methylenchlorid gelöst, mit Wasser versetzt und das Gemisch mit Kaliumhydrogencarbonat auf pH 8,0 gestellt. Die wässrige Phase wurde dreimal mit CH₂Cl₂ extrahiert und die organischen Phasen zweimal mit Wasser und einmal mit gesättiger NaCl-Lösung gewaschen, über Na₂SO₄ getrocknet und unter vermindertem Druck eingedampft. Man erhielt als Rückstand das o-Trifluormethyl-benzylbromid.
b) 40 ml Malonsäurediäthylester in 350 ml Aethylalkohol wurden portionsweise bei Raumtemperatur mit 18,6 g Natriumäthylat versetzt und dann innerhalb 30 Minuten mit 12 g o-Trifluormethyl-benzylbromid versetzt. Das Reaktionsgemisch wurde über Nacht bei Raumtemperatur gerührt, der Alkohol unter vermindertem Druck abdestilliert und der Rückstand in Aethylacetat gelöst. Die Lösung wurde zweimal mit Wasser und einmal mit NaCl-Lösung gewaschen, unter vermindertem Druck getrocknet und eingedampft. Der Rückstand wurde über 300 g SiO₂ mit CH₂Cl₂/AcOEt 95:5 chromatographiert und lieferte 11 g Diäthyl-[o-(trifluormethyl)benzyl]malonat als farbloses Oel.
c) 0,63 g Formamidinacetat in 40 ml abs. Aethylalkohol wurden bei Raumtemperatur mit 1,2 g Natriumäthylat versetzt, 30 Minuten bei Raumtemperatur gerührt und dann mit einer Lösung von 1,6 g Diäthyl-[o-(trifluormethyl)benzyl]malonat in 8 ml abs. Aethylalkohol bei Raumtemperatur tropfenweise versetzt. Nach 4 Stunden Rühren bei 50°C wurde das Reaktionsgemisch aufgearbeitet und lieferte 5-[o-(Trifluormethyl)benzyl]-4,6(1H,5H)pyrimidindion, Schmelzpunkt >290°C.
d) Aus 5-[o-(Trifluormethyl)benzyl]-4,6(1H,5H)pyrimidindion und Phosphoroxychlorid wurde das 5-[o-(Trifluormethyl)benzyl]-4,6-dichlorpyrimidin, Schmelzpunkt 60-63°C hergestellt.
e) 295 mg 4,6-Dichlor-5-[o-(trifluormethyl)benzyl]pyrimidin in 10 ml frisch destilliertem Dimethylsulfoxid wurden mit 342 mg α,α,α-Trifluor-p-toluolsulfonamid-mono-Kaliumsalz (aus dem entsprechenden Sulfonamid mit KOH und abs. Aethylalkohol) versetzt und 5 Stunden bei 150°C gerührt. Nach vollständiger Umsetzung wurde das Lösungsmittel unter vermindertem Druck abdestilliert, der Rückstand in Aethylacetat gelöst und die Lösung mit Kaliumbicarbonat-Lösung 10%ig, 0,5N HCl, Wasser und NaCl-Lösung gesättigt gewaschen. Die organische Phase wurde getrocknet und unter vermindertem Druck eingedampft. Der Rückstand wurde mittels Aethylacetat über 30 g SiO₂ chromatographiert und lieferte 135 mg N-[6-Chlor-6-[o-(trifluormethyl)benzyl]-4-pyrimidinyl]-α,α,α-trifluor-p-toluolsulfonamid als weissen Schaum. MS: 495 (M), 431 (-SO₂), 430 (-SO₂+H), 362 (-CF₃+SO₂).

### Beispiel 34

In Analogie zu Beispiel 33 wurde aus N-[6-Chlor-5-[o-(trifluormethyl)benzyl]-4-pyrimidinyl]-p-methoxybenzolsulfonamid und Aethylenglykol-Na das N-[6-(2-Hydroxyäthoxy)-5-[o-(trifluormethyl)benzyl]-4-pyrimidinyl]-p-methoxybenzolsulfonamid, Schmelzpunkt 100-107°C erhalten.

Das Ausgangsmaterial wurde wie folgt hergestellt:

In Analogie zu Beispiel 33, Abschnitt e), wurde aus 4,6-Dichlor-5-[o-(trifluormethyl)benzyl]pyrimidin und p-Methoxybenzolsulfonamid K-Salz das N-[6-Chlor-5-[o-(trifluormethyl)benzyl]-4-pyrimidinyl]-p-methoxybenzolsulfonamid als weisser Schaum, Schmelzpunkt 68-70°C erhalten.

### Beispiel 35

In Analogie zu Beispiel 33 wurde aus p-Chlor-N-[6-chlor-5-[o-(trifluormethyl)benzyl]-4-pyrimidinyl]benzolsulfonamid und Aethylenglykol-Na das p-Chlor-N-[6-(2-hydroxyäthoxy)-5-[o-(trifluormethyl)benzyl]-4-pyrimidinyl]benzolsulfonamid, Schmelzpunkt 134-135°C erhalten.

Das Ausgangsmaterial wurde in Analogie zu Beispiel 33, Abschnitt e) aus 4,6-Dichlor-5-[o-(trifluormethyl)benzyl]pyrimidin und p-Chlorbenzolsulfonamid K-Salz hergestellt; Schmelzpunkt >210°C (Zersetzung).

### Beispiel 36

In Analogie zu Beispiel 33 wurde aus N-[6-Chlor-5-(o-methoxybenzyl)-4-pyrimidinyl]-p-vinylbenzolsulfonanid und Aethylenglykol-Na das N-[6-(2-Hydroxyäthoxy)-5-(o-methoxybenzyl)-4-pyrimidinyl]-p-vinylbenzolsulfonamid, Schmelzpunkt 93-102°C erhalten.

Das Ausgangsmaterial wurde in Analogie zu Beispiel 33, Abschnitt e) aus 4,6-Dichlor-5-(o-methoxybenzyl)pyrimidin und p-Vinylbenzolsulfonamid K-Salz hergestellt; Schmelzpunkt 125-129°C.

### Beispiel 37

In Analogie zu Beispiel 33 wurde aus N-[6-Chlor-5-[o-(trifluormethyl)benzyl]-4-pyrimidinyl]-p-(methylthio)benzolsulfonamid und Aethylenglykol-Na das N-[6-(2-Hydroxyäthoxy)-5-[o-(trifluormethyl)benzyl]-4-pyrimidinyl]-p-(methylthio)benzolsulfonamid als gelbliches Harz erhalten.

Das Ausgangsmaterial wurde in Analogie zu Beispiel 33, Abschnitt e) aus 4,6-Dichlor-5-[o-(trifluormethyl)]benzylpyrimidin und p-(Methylthio)benzolsulfonamid hergestellt; IR: 3433 cm⁻¹ (NH); 1313 (SO₂); 1137 u. 1094 (F₃C).

### Beispiel 38

In Analogie zu Beispiel 32 wurde aus N-[6-Chlor-5-(2,4-dimethoxybenzyl)-4-pyrimidinyl]-p-isopropylbenzolsulfonamid, Aethylenglykol und Natrium das N-[5-(2,4-Dimethoxybenzyl)-6-(3-hydroxypropyl)-4-pyrimidinyl]-p-isopropylbenzolsulfonamid, Schmelzpunkt 97°C erhalten.

Das Ausgangsmaterial wurde wie folgt hergestellt:

In Analogie zu Beispiel 32, Abschnitt a) wurde aus 2,4-Dimethoxybenzaldehyd, Malonsäurediäthylester, Eisessig, Piperidin und Toluol das Diäthyl-(2,4-dimethoxybenzyliden)malonat hergestellt. Daraus wurde in Analogie zu Beispiel 32, Abschnitt b) das Diäthyl-(2,4-dimethoxybenzyl)malonat als klares Oel, Siedepunkt 160°C/0,4 mbar hergestellt.

In Analogie zu Beispiel 32, Abschnitt e) wurde aus Diäthyl-(2,4-dimethoxybenzyl)malonat, Formamidinacetat und dem Na-Salz von Aethanol das 5-(2,4-Dimethoxybenzyl)-4,6-pyrimidindiol und daraus in Analogie zu Beispiel 32, Abschnitt d) das 4,6-Dichlor-5-(2,4-dimethoxybenzyl)pyrimidin, Schmelzpunkt 130-131°C hergestellt.

In Analogie zu Beispiel 32, Abschnitt e) wurde schliesslich aus 4,6-Dichlor-5-(2,4-dimethoxybenzyl)pyrimidin, p-Isopropylbenzolsulfonamid-K (aus dem entsprechenden Sulfonamid mit Kalium-t-butylat in abs. MeOH) und DMSO das N-[6-Chlor-5-(2,4-dimethoxybenzyl)-4-pyrimidinyl]-p-isopropylbenzolsulfonamid, Schmelzpunkt 132-134°C hergestellt.

### Beispiel 39

Eine Lösung von 110 mg N-[6-(2-Hydroxyäthoxy)-5-(o-methoxybenzyl)-4-pyrimidinyl]-p-vinylbenzolsulfonamid in 3 ml abs. Tetrahydrofuran wurde mit 0,3 ml 3,4-Dihydro-2H-pyran und 4 Tropfen Trifluoressigsäure versetzt. Nach Kochen unter Rückfluss über Nacht wurde das Lösungsmittel unter vermindertem Druck abdestilliert, und der Rückstand mit Methylenchlorid/Aethylacetat (9:1) an Silicagel chromatographiert. Man erhielt 100 mg rac-N-[5-(o-Methoxybenzyl)-6-[2-[(tetrahydro-2H-pyran-2-yl)oxy]äthoxy]-4-pyrimidinyl]-p-vinylbenzolsulfonamid als weisses Harz, MS: 460 (M-SO₂+H); 430 (M-SO₂+OCH₃).

### Beispiel 40

Zu einem Gemisch von 1,5 ml Wasser und 4 ml Dioxan wurde bei Raumtemperatur der Reihe nach 318 mg rac-N-[5-(o-Methoxybenzyl)-6-[2-[(tetrahydro-2H-pyran-2-yl]oxy]äthoxy]-4-pyrimidinyl]-p-vinylbenzolsulfonamid, 5,3 mg Osmiumtetroxid und 270 mg Natrium(meta)perjodat. Nach 1 Stunde Rühren bei Raumtemperatur wurde das Dioxan unter vermindertem Druck abdestilliert, danach die wässrige Phase dreimal mit Aethylacetat extrahiert, das Aethylacetat zweimal mit Wasser und einmal mit NaCl-Lösung (gesättigt) gewaschen, getrocknet und unter vermindertem Druck abdestilliert. Der Rückstand wurde über 30 g SiO₂ mit CH₂Cl₂/Aethylacetat chromatographiert und lieferte 150 mg rac-p-[[5-(o-Methoxybenzyl)-6-[2-[(tedahydro-2H-pyran-2-yl)oxy]äthoxy]-4-pyrimidinyl]sulfamoyl]benzaldehyd als weissen Schaum. MS: 527 (M); 443 ( ); 432 (-OCH₃+SO₂).

### Beispiel 41

Zu einer aus 60 mg Magnesium und 0,15 ml Methyljodid in Diäthyläther hergestellten Grignard-Lösung wurden bei Raumtemperatur 170 mg rac-p-[[5-(o-Methoxybenzyl)-6-[2-[(tetrahydro-2H-pyran-2-yl]oxy]äthoxy]-4-pyrimidinyl]sulfamoyl]benzaldehyd und nach 30 Minuten 1 ml abs. Tetrahydrofuran zugegeben. Nach 3 Stunden Rühren bei Raumtemperatur wurde die Reaktion durch Zugabe von gesättigter Ammoniumchloridlösung abgebrochen, das Reaktionsgemisch mit Aethylacetat verdünnt und die wässrige Phase noch zweimal mit Aethylacetat extrahiert. Die organische Phase wurde mit Wasser und gesättigter NaCl-Lösung gewaschen, getrocknet und unter vermindertem Druck abdestilliert. Der Rückstand wurde über 35 g SiO₂ mit CH2Cl₂/Aethylacetat (8:2) und (1:1) chromatographiert und lieferte 135 mg p-[(RS)-1-Hydroxyäthyl]-N-[5-(-methoxybenzyl)-6-[2-[[(RS)-tetrahydro-2H-pyran-2-yl]oxy]äthoxy]-4-pyrimidinyl]benzolsulfonamid, Schmelzpunkt >56°C (Sublimation).

### Beispiel 42

53 mg rac-p-[[5-(o-Methoxybenzyl)-6-[2-[(tetrahydro-2H-pyran-2-yl]oxy]äthoxy]-4-pyrimidinyl]sulfamoyl]benzaldehyd wurden in 3 ml Methylalkohol gelöst und bei Raumtemperatur mit 37 mg Natriumborhydrid versetzt. Nach 1 Stunde Rühren bei Raumtemperatur wurden das Methanol bei vermindertem Druck abgedampft, der Rückstand in Aethylacetat gelöst, mit Wasser und NaCl-Lösung (gesättigt) gewaschen, getrocknet und unter vermindertem Druck abdestilliert. Man erhielt 42 mg rac-α-Hydroxy-N-[5-(o-methoxybenzyl)-6-[2-[(tetrahydro-2H-pyran-2-yl)oxy]äthoxy]-4-pyrimidinyl]-p-toluolsulfonamid als farbloses Oel. MS: 529 (M); 445 (Tetrahydro-2H-pyran-2-yl); 434 (-OCH₃+SO₂).

### Beispiel 43

53 mg rac-p-[[5-(o-Methoxybenzyl)-6-[2-[(tetrahydro-2H-pyran-2-yl]oxy]äthoxy]-4-pyrimidinyl]sulfamoyl]benzaldehyd wurden in 3 ml Aethylalkohol gelöst und bei Raumtemperatur mit 7 mg Hydroxylamin-hydrochlorid und 14 mg Kaliumcarbonat, fein pulv., versetzt. Nach 3 Stunden Rühren bei Raumtemperatur wurde das Aethanol unter vermindertem Druck abdestilliert, der Rückstand in Aethylacetat gelöst und mit Wasser und NaCl-Lösung (gesättigt) gewaschen. Die organische Phase wurde getrocknet und unter vermindertem Druck eingedampft, wobei rac-α-[(>E/Z)-Hydroxyimino-N-[5-(o-methoxybenzyl)-6-[2-[(tetrahydro-2H-pyran-2-yl)oxy]äthoxy]-4-pyrimidinyl]-p-toluolsulfonamid, Schmelzpunkt 49-52°C erhalten wurde.

### Beispiel 44

Eine Lösung von 60 mg p-[(RS-1-Hydroxyäthyl]-N-[5-(o-methoxybenql)-6-[2-[[(RS)-tetrahydro-2H-pyran-2-yl]oxy]äthoxy]benzolsulfonamid in 3 ml Tetrahydrofuran wurde mit 2 Tropfen 3N HCl versetzt. Nach 4 Stunden Rühren bei Raumtemperatur wurde das Reaktionsgemisch unter vermindertem Druck eingedampft. Der Rückstand wurde mit Methylenchlorid/Aethylacetat (1:1) und Aethylacetat an Silicagel chromatographiert und lieferte rac-N-6-(2-Hydroxyäthoxy)-5-(o-methoxybenzyl)-p-(1-hydroxyäthyl)benzolsulfonamid als weisses Harz. MS: 459 (M), 394 (-SO₂/H), 364 (-SO₂/OCH₃).

### Beispiel 45

In Analogie zu Beispiel 44 wurde aus rac-α-[(E/Z)-Hydroxyimino]-N-[5-(o-methoxybenzyl)-6-[2-[(tetrahydro-2H-pyran-2-yl)oxy]äthoxy]-4-pyrimidinyl]-p-toluolsulfonamid das α-[(E/Z)-Hydroxyimino]-N-[6-(2-hydroxyäthoxy)-5-(o-methoxybenzyl)-4-pyrimidinyl]-p-toluolsulfonamid als gelbes Harz erhalten. IR: 3403 und 3193 cm⁻¹ (W, OH), 2607 (W,NH); 1729 (W, C=N).

### Beispiel 46

In Analogie zu Beispiel 44 wurde aus rac-α-Hydroxy-N-[5-(o-methoxybenzyl)-6-[2-[(tetrahydro-2H-pyran-2-yl)oxy]äthoxy]-4-pyrimidinyl]-p-toluolsulfonamid das α-Hydroxy-N-[6-(2-hydroxyäthoxy)-5-(o-methoxybenzyl)-4-pyrimidinyl]-p-toluolsulfonamid als hellbraunes Harz erhalten. MS: 445 (M), 380 (-SO₂/H), 274.

### Beispiel 47

In Analogie zu Beispiel 44 wurde aus rac-p-[[5-(o-Methoxybenzyl)-6-[2-[(tetrahydro-2H-pyran-2-yl)oxy]äthoxy]-4-pyrimidinyl]sulfamoylbenzaldehyd der p-[[6-(2-Hydroxyäthoxy)-5-(o-methoxybenzyl)-4-pyrimidinyl]sulfamoyl]benzaldehyd als weisses Harz erhalten. MS: 443 (M), 348 (-SO₂/OCH₃), 274.

### Beispiel 48

208 mg N-[6-(2-Hydroxyäthoxy)-5-(o-methoxybenzyl)-4-pyrimidinyl]-p-vinylbenzolsulfonamid wurden in 3 ml abs. THF gelöst, mit 0,06 ml Pyridin und 0,07 ml Essigsäureanhydrid versetzt und 3 Stunden unter Rückfluss gekocht. Nach Abdestillieren des Lösungsmittels unter vermindertem Druck wurde der Rückstand in Aethylacetat gelöst, die Lösung mit Wasser und Kochsalzlösung gewaschen, getrocknet und eingedampft. Der Rückstand lieferte nach Chromatographie über Kieselgel mit Methylenchlorid und Methylenchlorid/Aethylacetat (19:1 und 9:1) das 2-[[5-(o-Methoxybenzyl)-6-[(p-vinylphenyl)sulfamoyl]-4-pyrimidinyl]oxy]äthylacetat als weisses Harz.

### Beispiel 49

In Analogie zu Beispiel 1 wurde aus N-[6-Chlor-5-(α,α,α-trifluor-p-tolyl)-4-pyrimidinyl]-p-isopropylbenzolsulfonamid und Aethylenglykol-Na das N-[6-(2-Hydroxyäthoxy)-5-(α,α,α-trifluor-p-tolyl)-4-pyrimidinyl]-p-isopropylbenzolsulfonamid, Schmelzpunkt 222-223°C (aus Aceton und Isopropyläther) erhalten.

Das Ausgangsmaterial wurde aus 4,6-Dichlor-5-α,α,α-trifluor-p-tolylpyrimidin und p-Isopropylbenzolsulfonamid hergestellt, Schmelzpunkt 266-269°C (aus Acetonitril).

### Beispiel 50

Zu einer Natriumglykolatlösung aus 46 mg Natrium in 1 ml Aethylenglykol wurden 190 mg N-[6-Chlor-5-(p-methoxyphenyl)-4-pydmidinyl]-p-toluolsulfonamid gegeben. Nach 5 Stunden Reaktionsdauer bei 100°C wurde das Reaktionsgemisch unter vermindertem Druck zur Trockene eingedampft, der Rückstand zwischen Aethylacetat und 1N Salzsäure verteilt, die organische Phase neutral gewaschen, getrocknet und unter vermindertem Druck eingedampft. Der Rückstand wurde mit Methylenchlorid und Aethylacetat (4:1 V/V) an Kieselgel chromatographiert. Man erhielt 175 mg N-[6-(2-Hydroxyäthoxy)-5-(p-methoxyphenyl)-4-pyrimidinyl]-p-toluolsulfonamid, Schmelzpunkt 147-149°C (aus Methylenchlorid/Hexan).

Das Ausgangsmaterial wurde wie folgt hergestellt:

Zu einer Lösung von 41,55 g p-Methoxyphenylessigsäure in 150 ml abs. Aethanol wurden 150 ml Orthoameisensäure-äthylester und 1 g Methansulfonsäure gegeben. Das Reaktionsgemisch wurde 20 Stunden auf 85°C erwärmt. Das gebildete Aethylformiat wurde fortlaufend aus dem Reaktionsgemisch abdestilliert. Danach wurde das Reaktionsgemisch mit Natriumäthylat neutralisiert, die Lösungsmittel abgedampft und der Rückstand in Methylenchlorid aufgenommen und destilliert. Man erhielt 46,7 g (p-Methoxy)phenylessigsäure-äthylester als farblose Flüssigkeit, Siedepunkt 84°C/0,025 Torr.

Zu 19,4 g des vorstehend erhaltenen Esters wurden 7,5 g Natriumäthylat und 120 ml Diäthylcarbonat gegeben. Die erhaltene Suspension wurde bei 130°C kräftig gerührt und das gebildete Aethanol aus dem Reaktionsgemisch abdestilliert. Danach wurde auf Raumtemperatur abgekühlt und das Reaktionsgemisch auf Eis und wässrige Salzsäure (10% Ueberschuss) gegossen. Nach Extraktion mit Aethylacetat und Aufarbeitung des Extrakts wurde das Produkt durch Destillation gereinigt. Man erhielt 25 g (p-Methoxy)phenylmalonsäure-diäthylester, Siedepunkt 115°C/0,05 Torr.

10,9 g Natriumäthylat wurden in 125 ml getrocknetem Aethanol suspendiert. Dazu wurden unter Eiskühlung 4,83 g Formamidinhydrochlorid und 13,3 g des im vorstehenden Absatz erhaltenen Malonesters zugegeben. Das Reaktionsgemisch wurde unter Feuchtigkeitsausschluss 3 Stunden bei Raumtemperatur gerührt, danach wurde das Lösungsmittel abgedampft, der Rückstand in 100 ml Wasser gelöst, die wässrige Phase mit Toluol gewaschen und angesäuert. Man erhielt 8 g 5-(p-Methoxy)phenyl-6-hydroxy-4(3H)-pyrimidinon, Schmelzpunkt >250°C.

1 g des im vorstehenden Absatz beschriebenen Pyrimidinons wurden in 5 ml Phosphoroxychlorid suspendiert. Die Suspension wurde unter Feuchtigkeitsausschluss bei 80°C gerührt, wobei eine klare Lösung erhalten wurde. Nach 30 Minuten wurde das überschüssige Reagens abdestilliert und der Rückstand in Methylenchlorid aufgenommen und mit wässriger Kaliumhydrogencarbonatlösung geschüttelt, bis keine Kohlensäureentwicklung mehr stattfand. Nach Abdampfen des Lösungsmittels wurde mit Methylenchlorid über Kieselgel filtriert. Man erhielt 0,7 g 4,6-Dichlor-5-(p-methoxyphenyl)pyrimidin, Schmelzpunkt 95-96°C.

Zu einer siedenden äthanolischen Kaliumhydroxidlösung (2 g Kaliumhydroxid 85%ig in 50 ml abs. Aethanol) wurden 5,15 g in Aethanol gelöstes p-Toluolsulfonamid gegeben. Danach wurden 50 ml abs. Benzol zugesetzt und der grösste Teil des Lösungsmittelgemisches wurde bei Normaldruck abdestilliert. Man erhielt 4,6 g p-Toluolsulfonamid-Kalium.

510 mg der im vorstehenden Absatz beschriebenen Dichlorpyrimidins und 840 mg p-Toluolsulfonamid-Kalium wurden in 3 ml trockenem Dimethylformamid gelöst. Die Lösung wurde 3 Stunden bei 120°C gehalten, danach wurde das Dimethylformamid abdestilliert, der Rückstand zwischen Aethylacetat und 1N Salzsäure verteilt, die organische Phase neutral gewaschen und eingedampft. Nach Zusatz von Methanol erhielt man 540 mg N-[6-Chlor-5-(p-methoxyphenyl)-4-pyrimidinyl]-p-toluolsulfonamid, Schmelzpunkt 210-212°C.

### Beispiel 51

In Analogie zu Beispiel 50 erhielt man aus 300 mg N-[6-Chlor-5-(p-methoxyphenyl)-4-pyrimidinyl]-p-methoxybenzolsulfonamid, 200 mg N-[6(2-Hydroxyäthoxy)-5-(p-methoxyphenyl)-4-pyrimidinyl]-p-methoxybenzolsulfonamid, Schmelzpunkt 132-134°C.

Das Ausgangsmaterial wurde wie folgt hergestellt:

Zu einer Lösung von 7,3 g p-Methoxybenzolsulfochlorid in 50 ml Tetrahydrofuran wurden unter Kühlung im Eisbad 25 ml 25%iges NH₄OH zugetropft. Anschliessend wurde das Reaktionsgemisch 30 Minuten bei 70°C (Badtemperatur) kräftig gerührt, danach wurde das Tetrahydrofuran abdestilliert. Der Rückstand wurde mit Aethylacetat extrahiert. Man erhielt p-Methoxybenzolsulfonamid, das wie in Beispiel 50 beschrieben in das Kaliumsalz übergeführt wurde.

Eine Lösung von 510 mg 4,6-Dichlor-5-(p-methoxyphenyl)pyrimidin und 680 mg p-Methoxybenzolsulfonamid-Kalium in 3 ml Dimethylformamid wurde 1 Stunde auf 130°C erwärmt. Nach Aufarbeiten des Reaktionsgemisches erhielt man 690 mg N-[6-Chlor-5-(p-methoxyphenyl)-4-pyrimidinyl]-p-methoxybenzolsulfonamid, Schmelzpunkt 165-167°C.

### Beispiel 52

In Analogie zu Beispiel 50 wurde aus N-[6-Chlor-5-(p-methoxyphenyl)-4-pyrimidinyl]-p-(methylthio)benzolsulfonamid das N-[6-(2-Hydroxyäthoxy)-5-(p-methoxyphenyl)-4-pyrimidinyl]-p-(methylthio)benzolsulfonamid, Schmelzpunkt 171-172°C erhalten.

Das Ausgangsmaterial wurde wie in Beispiel 50 beschrieben aus 4,6-Dichlor-5-(p-methoxy)phenylpyrimidin und (p-Methylthio)benzolsulfonamid-Kalium erhalten, Schmelzpunkt 204-205°C.

### Beispiel 53

In Analogie zu Beispiel 50 wurde aus N-[6-Chlor-5-(p-methoxyphenyl)-2-methyl-4-pyrimidinyl]-p-methoxybenzolsulfonamid das N-[6-(2-Hydroxyäthoxy)-5-(p-methoxyphenyl)-2-methyl-4-pyrimidinyl]-p-methoxybenzolsulfonamid erhalten, Schmelzpunkt 138-139°C.

Das Ausgangsmaterial wurde wie folgt hergestellt:

Zu einer Lösung von 5,6 g Natriummethylat in 75 ml abs. Aethanol wurden 2,94 g Acetamidinhydrochlorid und 6,9 g p-Methoxyphenylmalonsäure-diäthylester gegeben. Das Reaktionsgemisch wurde 3 Stunden unter Feuchtigkeitsausschluss bei Raumtemperatur und 1,5 Stunden bei 50°C gerührt. Danach wurde das Aethanol abdestilliert, der Rückstand in Wasser aufgenommen und die Suspension mit 5N Salzsäure angesäuert. Der Feststoff wurde abfiltriert und mit Wasser gewaschen, bis die Waschlösung ein pH 4,5 bis 5,7 erreichte. Das so erhaltene Produkte wurde mit Phosphoroxychlorid umgesetzt und lieferte 2,8 g 4,6-Dichlor-2-methyl-(p-methoxy)phenylpyrimidin, Schmelzpunkt 114-116°C. Umsetzung dieser Verbindung mit p-Methoxybenzolsulfonamid-Kalium lieferte N-[6-Chlor-5-(p-methoxyphenyl)-2-methyl-4-pyrimidinyl]-p-methoxybenzolsulfonamid, Schmelzpunkt 152-154°C.

### Beispiel 54

In Analogie zu Beispiel 50 wurden aus 615 mg N-[6-Chlor-5-(p-methoxyphenyl)-4-pyrimidinyl]-p-isopropylbenzolsulfonamid 550 mg N-[6-(2-Hydroxyäthoxy)-5-(p-methoxyphenyl)-4-pyrimidinyl]-p-isopropylbenzolsulfonamid erhalten, Schmelzpunkt 128-129°C.

Zur Ueberführung dieses Sulfonamids in das Natriumsalz wurden 87 mg in Methanol gelöst, die stöchiometrische Menge Natriummethylat zugegeben, das Lösungsmittel abdestilliert und Diisopropyläther zugesetzt.

Das Ausgangsmaterial wurde wie folgt hergestellt:

p-Isopropylbenzolsulfochlorid, Siedepunkt 105°C/0,25 Torr wurde aus Cumol hergestellt und in das entsprechende Sulfonamid übergeführt, Schmelzpunkt 104-105°C. Umsetzung von 765 mg 4,6-Dichlor-5-(p-methoxyphenyl)pyrimidin und 925 mg p-Isopropylbenzolsulfonamid-Kalium lieferte 720 mg N-[6-Chlor-5-(p-methoxyphenyl)-4-pyrimidinyl]-p-isopropylbenzolsulfonamid, Schmelzpunkt 181-182°C.

### Beispiel 55

In Analogie zu Beispiel 50 wurden aus 700 mg p-t-Butyl-N-[6-chlor-5-(p-methoxyphenyl)-4-pyrimidinyl]benzolsulfonamid 600 mg p-t-Butyl-N-[6-(2-hydroxyäthoxy)-5-(p-methoxyphenyl)-4-pyrimidinyl]benzolsulfonamid, Schmelzpunkt 165-166°C erhalten.

Das Ausgangsmaterial wurde aus p-t-Butylbenzolsulfonamid-Kalium und 4,6-Dichlor-5-(p-methoxyphenyl)pyrimidin erhalten. Schmelzpunkt 204-205°C.

### Beispiel 56

In Analogie zu Beispiel 50 wurden aus 216 mg rac-p-sek-Butyl-N-[6-chlor-5-(p-methoxyphenyl)-4-pyrimidinyl]benzolsulfonamid 185 mg rac-p-sek-Butyl-N-[6-(2-hydroxyäthoxy)-5-(p-methoxyphenyl)-4-pyrimidinyl]benzolsulfonamid, Schmelzpunkt 120-122°C erhalten.

Das Ausgangsmaterial wurde aus rac-p-sek-Butylbenzolsulfonamid-Kalium und 2,6-Dichlor-5-(p-methoxyphenyl)pyrimidin hergestellt, Schmelzpunkt 172-173°C.

### Beispiel 57

In Analogie zu Beispiel 50 wurden aus 280 mg N-[6-Chlor-5-[(p-methylthio)phenyl]-4-pyrimidinyl]-p-isopropylbenzolsulfonamid 240 mg N-[6-(2-Hydroxyäthoxy)-5-[p-(methylthio)phenyl]-4-pyrimidinyl]p-isopropylbenzolsulfonamid, Schmelzpunkt 135-136°C (aus Diisopropyläther) erhalten.

Das Ausgangsmaterial wurde wie folgt hergestellt:

15,2 g (p-Methylthio)benzaldehyd wurden in 50 ml Isopropanol gelöst. Zu dieser Lösung wurden unter Kühlung im Eisbad 1,31 g Natriumborhydrid in 150 ml Isopropanol innerhalb 0,5 Stunden getropft. Nach 1 Stunde Rühren bei Raumtemperatur wurden 5 ml Aceton zugesetzt, anschliessend wurde das Lösungsmittel abdestilliert. Der Rückstand wurde zwischen Methylenchlorid und Wasser verteilt. Nach Aufarbeiten wurde der (p-Methylthio)benzylalkohol, Schmelzpunkt 40-41°C (aus Isopropanol) erhalten.

7,71 g (p-Methylthio)benzylalkohol wurden in 25 ml trockenem Methylenchlorid gelöst. Unter Kühlung im Eisbad wurden dieser Lösung 4 ml SOCl₂ innerhalb von 30 Minuten zugegeben. Nach Abdestillieren des Lösungsmittels und des überschüssigen Reagenses wurde der Rückstand mit Methylenchlorid über Kieselgel filtriert. Nach Destillation wurden 4,3 g (p-Methylthio)benzylchlorid, Siedepunkt 92°C/0,05 Torr erhalten.

Zu einer Suspension von 10 g Kaliumcyanid und 0,1 g Natriumjodid in 100 ml Dimethylformamid wurden 9 g des im vorstehenden Absatz erhaltenen Benzylchlorids zugegeben. Das Reaktionsgemisch wurde unter Feuchtigkeitsausschluss 1 Stunde bei 90°C gerührt. Danach wurde das Dimethylformamid abdestilliert und der Rückstand zwischen Toluol und Wasser verteilt. Aufarbeiten der organischen Phase lieferte (p-Methylthio)benzylcyanid, Schmelzpunkt 28-30°C.

12 g (p-Methylthio)benzylcyanid wurden in 30 ml Aethylenglykol gelöst und mit 9 g NaOH (als 30%ige Lösung) versetzt. Das Reaktionsgemisch wurde 3 Stunden bei 140°C gerührt. Nach Abkühlen auf Raumtemperatur wurde mit 25%iger Salzsäure angesäuert, der Niederschlag in Aethylacetat aufgenommen und mit Wasser ausgeschüttelt. Man erhielt 11,5 g (p-Methylthio)phenylessigsäure; Schmelzpunkt 94-96°C.

11 g der vorstehend erhaltenen Säure wurden in 50 ml abs. Aethanol und 25 ml Orthoameisensäure-äthylester und 1 g Methansulfonsäure gelöst. Das bei der Reaktion gebildete Formiat wurde fortlaufend abdestilliert. Nach 4 Stunden war der Umsatz vollständig. Der saure Katalysator wurde mit der stöchiometrischen Menge Natriumäthylat neutralisiert, das Lösungsmittel abdestilliert, der Rückstand in Methylenchlorid aufgenommen und über Kieselgel filtriert. Man erhielt 12 g (p-Methylthio)phenylessigsäure-äthylester, Schmelzpunkt 46-47°C.

Die vorstehend erhaltene Verbindung wurde in Analogie zu dem in Beispiel 50 beschriebenen Verfahren in (p-Methylthio)phenylmalonsäurediäthylester übergeführt. Siedepunkt 120°C/0,05 Torr.

Aus dem vorstehend erhaltenen Malonsäure-diäthylester wurden in Analogie zu dem in Beispiel 50 beschriebene Verfahren 5-(p-Methylthio)phenyl-6-hydroxy-4(3H)-pyrimidinon erhalten.

Das vorstehend beschriebene Pyrimidinon wurde mit Natriummethylat in die Dialkoxyverbindung übergeführt, aus der durch Umsetzung mit Phosphoroxychlorid das 4,6-Dichlor-5-(p-methylthio)phenyl-pyrimidin erhaltene wurde.

Aus der vorstehend beschriebenen 4,6-Dichlor-Verbindung wurden durch Umsetzung mit p-Isopropylbenzolsulfonamid-Kalium das N-[6-Chlor-5-[p-(methylthio)phenyl[-4-pyrimidinyl]-p-isopropylbenzolsulfonamid, Schmelzpunkt 193-195°C erhalten.

### Beispiel 58

In Analogie zu Beispiel 50 wurden aus 230 mg N-[6-Chlor-5-[p-(methylthio)phenyl[-4-pyrimidinyl]-α,α,α-trifluor-p-toluolsulfonamid 160 mg N-[6-(2-Hydroxyäthoxy)-5-[p-(methylthio)phenyl]-4-pyrimidinyl]-α,α,α-trifluor-p-toluolsulfonamid, Schmelzpunkt 266-268°C erhalten.

Das Ausgangsmaterial wurde aus 4,6-Dichlor-5-p-(methylthio)phenylpyrimidin und α,α,α-Trifluor-p-toluolsulfonamid-Kalium erhalten, Schmelzpunkt 250-252°C.

### Beispiel 59

Zu einer Natriumglykolatlösung aus 1 ml trockenem Aethylenglykol und 46 mg Natrium wurden 300 mg N-[6-Chlor-5-(o-methoxybenzyl)-4-pyrimidinyl]-p-methoxybenzolsulfonamid gegeben. Das Reaktionsgemisch wurde 4 Stunden unter Argonatmosphäre auf 125°C erwärmt. Danach wurde das Aethylenglykol unter vermindertem Druck abdestilliert, der Rückstand zwischen Aethylacetat und 1N Salzsäure verteilt, die organische Phase neutral gewaschen, getrocknet und eingedampft. Der Rückstand wurde mit Methylenchlorid/Aethylacetat (1:1 V/V) an Kieselgel chromatographiert. Man erhielt 250 mg N-[6-(2-Hydroxyäthoxy)-5-(o-methoxybenzyl)-4-pyrimidinyl]-p-methoxybenzolsulfonamid, Schmelzpunkt 161-162°C.

Das Ausgangsmaterial wurde wie folgt hergestellt:

Durch Knoevenagel-Kondensation von o-Methoxybenzaldehyd mit Diäthylmalonat wurde o-Methoxybenzylidenmalonsäure-diäthylester erhalten, Siedepunkt 140°C/0,05 Torr.

Hydrierung der vorstehend erhaltenen Verbindung in Aethanol in Gegenwart von Palladium/Kohle lieferte o-Methoxybenzylmalonsäure-diäthylester, Siedepunkt 115°C/0,01 Torr.

Umsetzung von o-Methoxybenzylmalonsäure-diäthylester mit Formamidinhydrochlorid lieferte das 5-(o-Methoxybenzyl)-6-hydroxy-4(3H)-pyrimidinon aus dem durch Umsetzung mit Phosphoroxychlorid das 4,6-Dichlor-5-(o-methoxybenzyl)pyrimidin, Schmelzpunkt 95-96°C erhalten wurde.

Umsetzung von 4,6-Dichlor-5-(o-methoxybenzyl)-4-pyrimidin und p-Methoxybenzolsulfonamid-Kalium lieferte N-[6-Chlor-5-(o-methoxybenzyl)-4-pyrimidinyl]-p-methoxybenzolsulfonamid, Schmelzpunkt 149-151°C.

### Beispiel 60

In Analogie zu Beispiel 59 wurde aus N-[6-Chlor-5-(o-chlorbenzyl)-4-pyrimidinyl]-p-toluolsulfonamid das N-[6-(2-Hydroxyäthoxy)-5-(o-chlorbenzyl)-4-pyrimidinyl]-p-toluolsulfonamid erhalten.

Das Ausgangsmaterial wurde wie folgt hergestellt:

Malonsäure-diäthylester und o-Chlorbenzylchlorid wurden zu o-Chlorbenzylmalonsäure-diäthylester, Siedepunkt 115°C/0,05 Torr umgesetzt.

Kondensation des o-Chlorbenzylmalonsäure-diäthylesters mit Formamidin lieferte 5-(o-Chlorbenzyl)-6-hydroxy-4(3H)-pyrimidinon, das durch Umsetzung mit Phosphoroxychlorid 4,6-Chlor-5-(o-chlorbenzyl)pyrimidin, Schmelzpunkt 110-112°C lieferte.

Aus 4,6-Dichlor-5-(o-chlorbenzyl)pyrimidin und p-Toluolsulfonamid-Kalium wurde das N-[6-Chlor-5-(o-chlorbenzyl)-4-pyrimidinyl]-p-toluolsulfonamid erhalten, das als Rohprodukt eingesetzt wurde.

### Beispiel 61

In Analogie zu Beispiel 59 wurde aus N-[6-Chlor-5-(o-methoxybenzyl)-4-pyrimidinyl]-p-(methylthio)benzolsulfonamid das N-[6-(2-Hydroxyäthoxy)-5-(o-methoxybenzyl)-4-pyrimidinyl]-p-(methylthio)benzolsulfonamid, Schmelzpunkt 134-136°C erhalten.

Das Ausgangsmaterial wurde aus 4,6-Dichlor-5-(o-methoxybenzyl)pyrimidin und (p-Methylthio)benzolsulfonamid-Kalium hergestellt. Schmelzpunkt 157-159°C.

### Beispiel 62

In Analogie zu Beispiel 59 wurde aus N-[6-Chlor-5-(o-methoxybenzyl)-4-pyrimidinyl]-α,α,α-trifluor-p-toluolsulfonamid das N-[6-(2-Hydroxyäthoxy)-5-(o-methoxybenzyl)-4-pyrimidinyl]-α,α,α-trifluor-p-toluolsulfonamid, Schmelzpunkt 133-134°C erhalten.

Das Ausgangsmaterial wurde aus 4,6-Dichlor-5-(o-methoxybenzyl)pyrimidin und p-Trifluormethylbenzolsulfonamid-Kalium erhalten. Schmelzpunkt 163°C.

### Beispiel 63

In Analogie zu Beispiel 59 wurde aus N-[6-Chlor-5-(o-methoxybenzyl)-4-pyrimidinyl]-p-isopropylbenzolsulfonamid das N-[6-(2-Hydroxyäthoxy)-5-(o-methoxybenzyl)-4-pyrimidinyl]-p-isopropylbenzolsulfonamid, Schmelzpunkt 112-113°C erhalten.

Durch Behandlung mit Natriummethylat in Methanol wurde das Natriumsalz hergestellt, Schmelzpunkt 225°C.

Das Ausgangsmaterial wurde aus 4,6-Dichlor-5-(o-methoxybenzyl)pyrimidin und Isopropylbenzolsulfonamid-Kalium hergestellt, Schmelzpunkt 138-139°C.

### Beispiel 64

In Analogie zu Beispiel 59 wurde aus p-t-Butyl-N-[6-chlor-5-(o-methoxybenzyl)-4-pyrimidinyl]benzolsulfonamid das p-t-Butyl-N-[6-(2-hydroxyäthoxy)-5-(o-methoxybenzyl)-4-pyrimidinyl]benzolsulfonamid, Schmelzpunkt 138-140°C (aus Diisopropyläther) erhalten.

Das Ausgangsmaterial wurde aus 4,6-Dichlor-5-(o-methoxybenzyl)pyrimidin und p-t-Butylbenzolsulfonamid-Kalium hergestellt, Schmelzpunkt 215-216°C.

### Beispiel 65

In Analogie zu Beispiel 59 wurde aus N-[6-Chlor-5-(o-chlorbenzyl)-4-pyrimidinyl]-p-isopropylbenzolsulfonamid das N-[6-(2-Hydroxyäthoxy)-5-(o-chlorbenzyl)-4-pyrimidinyl]-p-isopropylbenzolsulfonamid erhalten.

Das Ausgangsmaterial wurde aus 4,6-Dichlor-5-(o-chlorbenzyl)pyrimidin und p-Isopropylbenzolsulfonamid-Kalium hergestellt, Schmelzpunkt 166-167°C.

### Beispiel 66

In Analogie zu Beispiel 59 wurde aus N-[6-Chlor-5-(o-(methylthio)benzyl)-4-pyrimidinyl]-p-isopropylbenzolsulfonamid das N-[6-(2-Hydroxyäthoxy)-5-(o-(methylthio)benzyl)-4-pyrimidinyl]-p-isopropylbenzolsulfonamid erhalten.

Das Ausgangsmaterial wurde wie folgt hergestellt:

Durch Umsetzung von Thiosalicylsäure mit Dimethylsulfat in Gegenwart von Tetrabutylammoniumbromid wurde 2-(Methylthio)benzoesäuremethylester, Schmelzpunkt 64°C hergestellt. Reduktion mit Lithiumaluminiumhydrid in trockenem Tetrahydrofuran lieferte 2-(Methylthio)benzylalkohol, der durch Umsetzung mit SOCl₂ in 2-(Methylthio)benzylchlorid, Siedepunkt 90°C/0,3 Torr übergeführt wurde. Umsetzung von Malonsäurediäthylester mit 2-(Methylthio)benzylchlorid lieferte 2-(Methylthio)benzylmalonsäure-diäthylester, Siedepunkt 130°C/0,05 Torr. Kondensation mit Formamidin lieferte 5-[o-(Methylthio)benzyl]-6-hydroxy-4(3H)-pyrimidinon, das in 4,6-Dichlor-5-[o-(methylthio)benzyl]pyrimidin, Schmelzpunkt 91°C übergeführt wurde. Aus 4,6-Dichlor-5-(o-(methylthio)benzyl)pyrimidin und p-Isopropylbenzolsulfonamid-Kalium wurde schliesslich N-[6-Chlor-5-(o-(methylthio)benzyl)-4-pyrimidinyl]-p-isopropylbenzolsulfonamid, Schmelzpunkt 145-146°C erhalten.

### Beispiel 67

In Analogie zu Beispiel 59 wurde aus N-[6-Chlor-5-(o-chlorbenzyl)-4-pyrimidinyl]-p-isobutylbenzolsulfonamid das N-[6-(2-Hydroxyäthoxy)-5-(o-chlorbenzyl)-4-pyrimidinyl]-p-isobutylbenzolsulfonamid, Schmelzpunkt 130-131°C erhalten.

Das Ausgangsmaterial wurde aus 4,6-Dichlor-5-(o-chlorbenzyl)pyrimidin und p-Isobutylbenzolsulfonamid-Kalium hergestellt, Schmelzpunkt 147-149°C.

### Beispiel 69

In Analogie zu Beispiel 50 wurde aus N-[6-Chlor-5-(o-chlorbenzyl)-4-pyrimidinyl]-p-cyclohexylbenzolsulfonamid das N-[6-(2-Hydroxyäthoxy)-5-(o-chlorbenzyl)-4-pyrimidinyl]-p-cyclohexylbenzolsulfonamid, Schmelzpunkt 164-165°C erhalten.

Das Ausgangsmaterial wurde aus 4,6-Dichlor-5-(o-chlorbenzyl)pyrimidin und p-Cyclohexylbenzolsulfonamid-Kalium hergestellt, Schmelzpunkt 107-108°C.

### Beispiel 70

In Analogie zu Beispiel 59 wurde aus N-[6-Chlor-5-(o-chlorbenzyl)-4-pyrimidinyl]-p-isopentylbenzolsulfonamid das N-[6-(2-Hydroxyäthoxy)-5-(o-chlorbenzyl)-4-pyrimidinyl]-p-isopentylbenzolsulfonamid, Schmelzpunkt 127-128°C (aus Diisopropyläther) erhalten.

Das Ausgangsmaterial wurde aus 4,6-Dichlor-5-(o-chlorbenzyl)pyrimidin und p-Isopentylbenzolsulfonamid-Kalium hergestellt, Schmelzpunkt 139-140°C.

### Beispiel 71

In Analogie zu Beispiel 59 wurde aus N-[6-Chlor-5-(o-methoxybenzyl)-4-pyrimidinyl]-p-(isopropylthio)benzolsulfonamid das N-[6-(2-Hydroxyäthoxy)-5-(o-methoxybenzyl)-4-pyrimidinyl]-p-(isopropylthio)benzolsulfonamid, Schmelzpunkt 127-128°C (aus Diisopropyläther) erhalten.

Das Ausgangsmaterial wurde aus 4,6-Dichlor-5-(o-methoxybenzyl)pyrimidin und p-(Isopropylthio)benzolsulfonamid-Kalium hergestellt.

### Beispiel 72

In Analogie zu Beispiel 1 wurde aus p-Chlor-N-[6-chlor-5-(p-chlorphenyl)-2-methyl-4-pyrimidinyl]benzolsufonamid und Aethylenglykol-Na das p-Chlor-N-[5-(p-chlorphenyl)-6-(2-hydroxyäthoxy)-2-methyl-4-pyrimidinyl]benzolsulfonamid, Schmelzpunkt 163-164°C (aus Aether) erhalten.

Das Ausgangsmaterial wurde wie folgt hergestellt:

Aus Diäthyl-p-chlorphenylmalonat, Acetamidin-hydrochlorid und Natriummethylat wurde das 5-(p-Chlorphenyl)-2-methyl-4,6(1H,5H)-pyrimidin-dion, Schmelzpunkt >270°C und daraus mit POCl₃ das 4,6-Dichlor-5-(p-chlorphenyl)-2-methylpyrimidin, Schmelzpunkt 181-183°C (aus Methylenchlorid und Isopropyläther) hergestellt.

Umsetzung dieser Verbindung mit p-Chlorphenylsulfonamid lieferte p-Chlor-N-[6-chlor-5-(p-chlorphenyl)-2-methyl-4-pyrimidinyl]benzolsulfonamid, Schmelzpunkt 196-197°C (aus Acetonitril).

### Beispiel 73

In Analogie zu Beispiel 1 wurde aus p-Chlor-N-[6-chlor-5-(p-nitrophenyl)-4-pyrimidinyl]benzolsulfonamid und p-Chlorphenylsulfonamid das p-Chlor-N-[6-(2-hydroxyäthoxy)-5-(p-nitrophenyl)-4-pyrimidinyl]benzolsulfonamid, Schmelzpunkt 223-225°C (aus Methylenchlorid und Isopropyläther) erhalten.

Das Ausgangsmaterial wurde wie folgt hergestellt:

3,5 g Diäthyl-p-nitrophenylmalonat und 1,6 g Formamidinacetat wurde 3 Stunden auf 100°C erwärmt. Danach wurden noch 3,2 g Formamidinacetat, 5 ml abs. Dimethylformamid und 1 ml Eisessig zugegeben und das Reaktionsgemisch 16 Stunden auf 110°C erwärmt. Nach Eindampfen des Lösungsmittels unter vermindertem Druck, wurde der Rückstand mit Aether verrieben, abgenutscht und in einer 1N NaOH-Lösung aufgenommen. Die Lösung wurde mit etwas Kohle versetzt, abfiltriert und mit Eisessig auf pH = 4,5 eingestellt. Der Niederschlag wurde unter vermindertem Druck bei 80°C getrocknet und, danach in 20 ml POCl₃ und 1 ml Dimethylanilin aufgenommen und unter Rühren 3 Stunden am Rückfluss gekocht. Nach dem Eindampfen des Lösungsmittels unter vermindertem Druck wurde der Rückstand in Essigester aufgenommen, die organische Lösung mit kaltem Wasser gewaschen, getrocknet und eingedampft. Der Rückstand wurde auf Kieselgel mit Cyclohexan-Aether 9:1 chromatographiert und lieferte 4,6-Dichlor-5-(p-nitrophenyl)pyrimidin, Schmelzpunkt 159-161°C (aus Isopropyläther).

Umsetzung dieser Verbindung mit p-Chlorphenylsulfonamid lieferte p-Chlor-N-[6-chlor-5-(p-nitrophenyl)-4-pyrimidinyl]benzolsulfonamid, Schmelzpunkt 282-285°C (aus Acetonitril).

### Beispiel 74

200 mg p-Chlor-N-[6-(2-hydroxyäthoxy)-5-(p-nitrophenyl)-4-pyrimidinyl]benzolsulfonamid wurden in 15 ml Eisessig und 2 ml 4N HCl in Dioxan über 50 mg Palladiumkohle (10%) bei Raumtemperatur und normalem Druck hydriert. Nach dem Abnutschen vom Katalysator wurde die Lösung unter vermindertem Druck eingedampft, der Rückstand in 30 ml Methanol gelöst und die Lösung mit 1 ml Dioxan-HCl versetzt. Nach 16 Stunden wurde die Lösung unter vermindertem Druck eingedampft und der Rückstand aus Methanol und Acetonitril umkristallisiert. Man erhielt N-[5-(p-Aminophenyl)-6-(2-hydroxyäthoxy)-4-pyrimidinyl]-p-chlorbenzolsulfonamid-hydro-Chlorid, Schmelzpunkt 206°C (unter Zersetzung).

### Beispiel 75

In Analogie zu Beispiel 1 wurde aus p-Chlor-N-[5-(4-biphenylyl)-6-chlor-4-pyrimidinyl]benzolsulfonamid und Aethylenglykol-Na das N-[5-(4-Biphenylyl)-6-(2-hydroxyäthoxy)-4-pyrimidinyl]-p-chlorbenzolsulfonamid, Schmelzpunkt 213-214°C (aus Essigester) erhalten.

Das Ausgangsmaterial wurde wie folgt hergestellt:

Aus Diäthyl-4-biphenylmalonat, Formamidinacetat und Natriummethylat wurde das 5-(4-Biphenylyl)-4,6(1H,5H)-pyrimidindion, Schmelzpunkt >280°C, und daraus mit POCl₃ das 5-(4-Biphenylyl)-4,6-dichlorpyrimidin, Schmelzpunkt 144°C (aus Methylenchlorid und n-Hexan) erhalten.

Umsetzung dieser Verbindung mit p-Chlorphenylsulfonamid lieferte p-Chlor-N-[5-(4-biphenylyl)-6-chlor-4-pyrimidinyl]benzolsulfonamid, Schmelzpunkt 234-235°C (aus Acetonitril).

### Beispiel 76

In Analogie zu Beispiel 1 wurde aus p-Chlor-N-(6-chlor-5-(α,α,α-trifluor-p-tolyl)-4-pyrimidinyl]benzolsulfonamid und Aethylenglykol-Na das p-Chlor-N-[(6-hydroxyäthoxy)-5-(α,α,α-trifluor-p-tolyl)-4-pyrimidinyl]benzolsulfonamid, Schmelzpunkt 213-124°C (aus Aceton und Isopropyläther) erhalten.

Das Ausgangsmaterial wurde wie folgt hergestellt:

Aus α,α,α-Trifluor-p-tolyl-malonat und Formamidinacetat wurde das 5-α,α,α-Trifluor-p-tolyl)-4,6(1H,5H)-pyrimidindion, Schmelzpunkt >280°C, und daraus mit POCl₃ das 4,6 Dichlor-5-(α,α,α-p-tolyl)-pyrimidin, Schmelzpunkt 94-95°C (aus n-Hexan) erhalten.

Umsetzung dieser Verbindung mit p-Chlorphenylsulfonamid lieferte p-Chlor-N-(6-chlor-5-(α,α,α-trifluor-p-tolyl)-4-pyrimidinyl]benzolsulfonamid, Schmelzpunkt 262-264°C (aus Acetonitril).

### Beispiel 77

In Analogie zu Beispiel 27 wurde aus N-[5-[p-(Benzyloxy)phenyl]-6-(2-hydroxyäthoxy)-4-pyrimidinyl]-p-chlorbenzolsufonamid das p-Chlor-N-[5-(p-hydroxyphenyl)-6-(2-hydroxyäthoxy)-4-pyrimidinyl]benzolsulfonamid, Schmelzpunkt 207-209°C (aus Acetonitril und Isopropyläther) erhalten.

### Beispiel 78

In Analogie zu Beispiel 1 wurde aus N-[5-[p-(Benzyloxy)phenyl]-6-chlor-4-pyrimidinyl]-p-chlorbenzolsulfonamid und Aethylenglykol-Na das N-[5-[p-(Benzyloxy)phenyl]-6-(2-hydroxyäthoxy)-4-pyrimidinyl]-p-chlorbenzolsulfonamid, Schmelzpunkt 160-161°C (aus Aether) erhalten.

Das Ausgangsmaterial wurde wie folgt hergestellt:

Aus Diäthyl-[p-(benzyloxy)phenyl]malonat und Formamidinacetat wurde das 5-[p-(Benzyloxy)phenyl]-4,6(1H,5H)-dion, >280°C, und daraus mit POCl₃ das 5-[p-(Benzyloxy)phenyl]-4,6-dichlorpyrimidin, Schmelzpunkt 115-116°C (aus Methylenchlorid und Isopropyläther) erhalten. Umsetzung dieser Verbindung mit p-Chlorphenylsulfonamid lieferte N-[5-[p-(Benzyloxy)phenyl]-6-chlor-4-pyrimidinyl]-p-chlorbenzolsulfonamid, Schmelzpunkt 234-236°C (aus Essigester).

### Beispiel 79

In Analogie zu Beispiel 1 wurde aus N-(6-Chlor-4-(α,α,α-trifluor-p-tolyl)-4-pyrimidinyl]-α,α,α-trifluor-p-toluolsulfonamid und Aethylenglykol-Na das N-[6-(2-Hydroxyäthoxy)-5-(α,α,α-trifluor-p-tolyl)-4-pyrimidinyl] -α,α,α-trifluor-p-toluolsulfonamid, Schmelzpunkt 165-166°C (aus Methylenchlorid und Isopropyläther) erhalten.

Das Ausgangsmaterial wurde aus 4,6-Dichlor-5-α,α,α-p-tolyl)-pyrimidin und α,α,α-Trifluor-p-tolylsulfonamid hergestellt, Schmelzpunkt >270°C (aus Acetonitril).

### Beispiel 80

In Analogie zu Beispiel 1 wurde aus p-Chlor-N-[6-chlor-5-(2-naphthylmethyl)-4-pyrimidinyl]benzolsulfonamid und Aethylenglykol-Na das p-Chlor-N-[6-(2-hydroxyäthoxy)-5-(2-naphthylmethyl)-4-pyrimidinyl]benzolsulfonamid, Schmelzpunkt 161°C (aus Acetonitril und Isopropyläther) erhalten.

Das Ausgangsmaterial wurde wie folgt hergestellt:

Aus Diäthyl-(2-naphthylmethyl)malonat und Formamidinacetat wurde das 5-(2-Naphthylmethyl)-4,6(1H,5H)-pyrimidindion, Schmelzpunkt >270°C und daraus mit POCl₃ das 4,6-Dichlor-5-(2-naphthylmethyl)pyrimidin, Schmelzpunkt 161-162°C (aus Methylenchlorid und Isopropyläther) erhalten.

Umsetzung dieser Verbindung mit p-Chlorphenylsulfonamid lieferte p-Chlor-N-[6-chlor-5-(2-naphthylmethyl)-4-pyrimidinyl]benzolsulfonamid, Schmelzpunkt 197-199°C (aus Acetonitril).

### Beispiel 81

In Analogie zu Beispiel 1 wurde aus N-[5-(p-Bromphenyl)-6-chlor-4-pyrimidinyl]-p-isopropylbenzolsulfonamid und Aethylenglykol-Na das N-[5-(p-Bromphenyl)-6-(2-hydroxyäthoxy)-4-pyrimidinyl]-p-isopropylbenzolsulfonamid, Schmelzpunkt 207-208°C (aus Acetonitril und Isopropyläther) erhalten.

Das Ausgangsmaterial wurde aus 5-(p-Bromphenyl)-4,6-dichlorpyrimidin und p-Isopropylbenzolsulfonamid hergestellt, Schmelzpunkt 271-273°C (aus Acetonitril).

### Beispiel 82

In Analogie zu Beispiel 1 wurde aus N-[6-Chlor-5-(p-chlorphenyl)-4-pyrimidinyl]-p-isopropyl-benzolsulfonamid und Aethylenglykol-Na das N-[5-(p-Chlorphenyl)-6-(2-hydroxyäthoxy)-4-pyrimidinyl]-p-isopropylbenzolsulfonamid, Schmelzpunkt 162-164°C (aus Acetonitril und Isopropyläther) erhalten.

Das Ausgangsmaterial wurde aus 4,6-Dichlor-5-(p-chlorphenyl)pyrimidin und p-Isopropylbenzolsulfonamid hergestellt, Schmelzpunkt 266-268°C (aus Acetonitril).

### Beispiel 83

In Analogie zu Beispiel 1 wurde aus N-(6-Chlor-5-p-tolyl-4-pyrimidinyl)-p-isopropylbenzolsulfonamid und Aethylenglykol-Na das N-[6-Hydroxyäthoxy)-5-p-tolyl-4-pyrimidinyl]-p-isopropylbenzolsulfonamid, Schmelzpunkt 142-144°C (aus Isopropyläther) erhalten.

Mit Natriummethylat wurde daraus das Na-Salz, amorphe Substanz, hergestellt.

Das Ausgangsmaterial, N-(6-Chlor-5-p-tolyl-4-pyrimidinyl)-p-isopropylbenzolsulfonamid, Schmelzpunkt 211-213°C (aus Acetonitril) wurde aus 4,6-Dichlor-5-p-tolylpyrimidin und p-Isopropylbenzolsulfonamid hergestellt.

### Beispiel 84

In Analogie zu Beispiel 1 wurde aus p-tert-Butyl-N-(6-chlor-5-p-tolyl-4-pyrimidinyl)benzolsulfonamid und Aethylenglykol-Na das p-tert-Butyl-N-[6-(2-hydroxyäthoxy)-5-p-tolyl-4-pyrimidinyl]benzolsulfonamid, Schmelzpunkt 169-170°C (aus Isopropyläther) und daraus mit Natriummethylat das amorphe Na-Salz erhalten.

Das Ausgangsmaterial wurde aus 4,6-Dichlor-5-p-tolylpyrimidin und p-tert-Butylbenzolsulfonamid hergestellt, Schmelzpunkt 222-224°C (aus Acetonitril).

### Beispiel 85

In Analogie zu Beispiel 1 wurde aus N-(6-Chlor-5-p-tolyl-4-pyrimidinyl)-p-(2-methoxyäthoxy)benzolsulfonamid und Aethylenglykol-Na das N-[6-(2-Hydroxyäthoxy)-5-p-tolyl-4-pyrimidinyl]-p-(2-methoxyäthoxy)benzolsulfonamid, Schmelzpunkt 155-156°C (aus Isopropyläther) erhalten.

Das Ausgangsmaterial wurde aus 4,6-Dichlor-5-p-tolylpyrimidin und p-(2-Methoxyäthoxy)benzolsulfonamid hergestellt, Schmelzpunkt 172-173°C (aus Methylenchlorid und Isopropyläther).

### Beispiel 86

In Analogie zu Beispiel 1 wurde aus N-(6-Chlor-5-p-tolyl-4-pyrimidinyl)-p-(trifluormethoxy)benzolsulfonamid und Aethylenglykol-Na das N-[6-(2-Hydroxyäthoxy)-5-p-tolyl-4-pyrimidinyl]-p-(trifluormethoxy)benzolsulfonamid, Schmelzpunkt 147-148°C (aus Isopropyläther) erhalten.

Das Ausgangsmaterial wurde aus 4,6-Dichlor-5-p-tolylpyrimidin und p-(Trifluormethoxy)benzolsulfonamid hergestellt, Schmelzpunkt 205-206°C (aus Acetonitril und Isopropyläther).

### Beispiel 87

In Analogie zu Beispiel 1 wurde aus p-Butyl-N-(6-chlor-5-p-tolyl-4-pyrimidinyl)benzolsulfonamid und Aethylenglykol-Na das p-Butyl-N-[6-(2-hydroxyäthoxy)-5-p-tolyl-4-pyrimidinyl]benzolsulfonamid, Schmelzpunkt 136-137°C (aus Isopropyläther) erhalten.

Das Ausgangsmaterial wurde aus 4,6-Dichlor-5-p-tolylpyrimidin und p-Butylbenzolsulfonamid hergestellt, Schmelzpunkt 168-169°C (aus Acetonitril und Isopropyläther).

### Beispiel 88

In Analogie zu Beispiel 1 wurde aus N-(6-Chlor-5-p-tolyl-4-pyrimidinyl)-2-naphthalinsulfonamid und Aethylenglykol-Na das N-[6-(2-Hydroxyäthoxy)-5-p-tolyl-4-pyrimidinyl]-2-naphthalinsulfonamid, Schmelzpunkt 161-162°C (aus Aceton und Isopropyläther) erhalten.

Das Ausgangsmaterial wurde aus 4,6-Dichlor-5-p-tolylpyrimidin und 2-Naphthalinsulfonamid hergestellt, Schmelzpunkt 198-202°C (aus Acetonitril).

### Beispiel 89

In Analogie zu Beispiel 1 wurde aus N-(6-Chlor-5-p-tolyl-4-pyrimidinyl)-p-toluolsulfonamid und Aethylenglykol-Na das N-[6-(2-Hydroxyäthoxy)-5-p-tolyl-4-pyrimidinyl]-p-toluolsulfonamid, Schmelzpunkt 169-170°C (aus Aceton und Isopropyläther) erhalten.

Das Ausgangsmaterial wurde aus 4,6-Dichlor-5-p-tolylpyrimidin und p-Toluolsulfonamid hergestellt, Schmelzpunkt 213-214°C (aus Acetonitril und Isopropyläther).

### Beispiel 90

In Analogie zu Beispiel 1 wurde aus N-(6-Chlor-5-p-tolyl-4-pyrimidinyl)-α,α,α-trifluor-p-toluolsulfonamid und Aethylenglykol-Na das N-[6-(2-Hydroxyäthoxy)-5-p-tolyl-4-pyrimidinyl]-α,α,α-trifluor-p-toluolsulfonamid, Schmelzpunkt 162-163°C (aus Acetonitril und Isopropyläther) erhalten.

Das Ausgangsmaterial wurde aus 4,6-Dichlor-5-p-tolylpyrimidin und α,α,α-Trifluor-p-toluolsulfonamid hergestellt, Schmelzpunkt 231-233°C (aus Acetonitril).

### Beispiel 91

In Analogie zu Beispiel 1 wurde aus N-(6-Chlor-5-p-tolyl-4-pyrimidinyl)-p-fluorbenzolsulfonamid und Aethylenglykol-Na nach Chromatographie das p-Fluor-N-[6-(2-hydroxyäthoxy)-5-p-tolyl-4-pyrimidinyl]benzolsulfonamid, Schmelzpunkt 167-168°C (aus Aceton und Isopropyläther) und das p-(2-Hydroxyäthoxy)-N-[6-(2-hydroxyäthoxy)-5-p-tolyl-4-pyrimidinyl]benzolsulfonamid, Schmelzpunkt 174-176°C (aus Aceton und Isopropyläther) erhalten.

Das Ausgangsmaterial wurde aus 4,6-Dichlor-5-p-tolylpyrimidin und p-Fluorbenzolsulfonamid hergestellt, Schmelzpunkt 207-208°C (aus Acetonitril und Isopropyläther).

### Beispiel 92

In Analogie zu Beispiel 1 wurde aus N-(6-Chlor-5-p-tolyl-4-pyrimidinyl)-p-propylbenzolsulfonamid und Aethylenglykol-Na das N-[6-(2-Hydroxyäthoxy)-5-p-tolyl-4-pyrimidinyl]-p-propylbenzolsulfonamid, Schmelzpunkt 152-153°C (aus Isopropyläther) erhalten.

Das Ausgangsmaterial wurde aus 4,6-Dichlor-5-p-tolylpyrimidin und p-Propylbenzolsulfonamid hergestellt, Schmelzpunkt 171-172°C (aus Acetonitril).

### Beispiel 93

In Analogie zu Beispiel 1 wurde aus N-(6-Chlor-5-p-tolyl-4-pyrimidinyl)-o-propylbenzolsulfonamid und Aethylenglykol-Na das N-[6-(2-Hydroxyäthoxy)-5-p-tolyl-4-pyrimidinyl]-o-propylbenzolsulfonamid,Schmelzpunkt 195-196°C (aus Methylenchlorid und Isopropyläther) erhalten.

Das Ausgangsmaterial wurde aus 4,6-Dichlor-5-p-tolylpyrimidin und o-Propylbenzolsulfonamid hergestellt, Schmelzpunkt 150-151°C (aus Isopropyläther).

### Beispiel 94

In Analogie zu Beispiel 1 wurde aus N-(6-Chlor-5-p-tolyl-4-pyrimidinyl)-p-äthylbenzolsulfonamid und Aethylenglykol-Na das p-Aethyl-N-[6-(2-hydroxyäthoxy)-5-p-tolyl-4-pyrimidinyl]benzolsulfonamid, Schmelzpunkt 138-139°C (aus Methylenchlorid und Isopropyläther) erhalten.

Das Ausgangsmaterial wurde aus 4,6-Dichlor-5-p-tolylpyrimidin und p-Aethylbenzolsulfonamid hergestellt, Schmelzpunkt 180-181°C (aus Acetonitril).

### Beispiel 95

In Analogie zu Beispiel 1 wurde aus N-(6-Chlor-5-p-tolyl-4-pyrimidinyl)-o-äthylbenzolsulfonamid und Aethylenglykol-Na das o-Aethyl-N-[6-(2-hydroxyäthoxy)-5-p-tolyl-4-pyrimidinyl]benzolsulfonamid, Schmelzpunkt 136-138°C (aus Aceton und Isopropyläther) erhalten.

Das Ausgangsmaterial wurde aus 4,6-Dichlor-5-p-tolylpyrimidin und o-Aethylbenzolsulfonamid hergestellt, Schmelzpunkt 159-160°C (aus Acetonitril und Isopropyläther).

### Beispiel 96

In Analogie zu Beispiel 1 wurde aus N-(6-Chlor-5-p-tolyl-4-pyrimidinyl)-p-cyclopentylbenzolsulfonamid und Aethylenglykol-Na das p-Cyclopentyl-N-[6-(2-hydroxyäthoxy)-5-p-tolyl]benzolsulfonamid, Schmelzpunkt 179-181°C (aus Aceton und Isopropyläther) erhalten.

Das Ausgangsmaterial wurde aus 4,6-Dichlor-5-p-tolylpyrimidin und p-Cyclopentylbenzolsulfonamid hergestellt, Schmelzpunkt 192-194°C (aus Acetonitril und Isopropyläther).

### Beispiel 97

In Analogie zu Beispiel 1 wurde aus N-(6-Chlor-5-p-tolyl-4-pyrimidinyl)-α,α,α-trifluor-o-toluolsulfonamid und Aethylenglykol-Na das α,α,α-TrifluorN-[6-(2-hydroxyäthoxy)-5-p-tolyl-4-pyrimidinyl]-o-toluolsulfonamid, Schmelzpunkt 166-167°C (aus Methylenchlorid und Isopropyläther) erhalten.

Das Ausgangsmaterial wurde aus 4,6-Dichlor-5-p-tolylpyrimidin und α,α,α-Trifluor-o-toluolsulfonamid hergestellt, Schmelzpunkt 129-131°C (aus Methylenchlorid und Isopropyläther).

### Beispiel 98

In Analogie zu Beispiel 1 wurde aus N-(6-Chlor-5-p-tolyl-4-pyrimidinyl)-o-toluolsulfonamid und Aethylenglykol-Na das N-[6-(2-Hydroxyäthoxy)-5-p-tolyl-4-pyrimidinyl]-o-toluolsulfonamid, Schmelzpunkt 149-150°C (aus Essigester und Isopropyläther) erhalten.

Das Ausgangsmaterial wurde aus 4,6-Dichlor-5-p-tolylpyrimidin und o-Toluolsulfonamid hergestellt, Schmelzpunkt 198-199°C (aus Acetonitril).

### Beispiel 99

In Analogie zu Beispiel 1 wurde aus N-(6-Chlor-5-p-tolyl-4-pyrimidinyl)-2,4-xylolsulfonamid und Aethylenglykol-Na das N-[6-(2-Hydroxyäthoxy)-5-p-tolyl-4-pyrimidinyl]-2,4-xylolsulfonamid, Schmelzpunkt 158-159°C (aus Isopropyläther) erhalten.

Das Ausgangsmaterial wurde aus 4,6-Dichlor-5-p-tolylpyrimidin und 2,4-Xylolsulfonamid hergestellt, Schmelzpunkt 233°C (aus Acetonitril und Isopropyläther).

### Beispiel 100

In Analogie zu Beispiel 1 wurde aus p-Chlor-N-[6-chlor-5-(1-naphthylmethyl)-4-pyrimidinyl]benzolsulfonamid und Aethylenglykol-Na das p-Chlor-N-[6-(2-hydroxyäthoxy)-5-(1-naphthylmethyl)-4-pyrimidinyl]benzolsulfonamid, Schmelzpunkt 204-205°C (aus Acetonitril und Isopropyläther) erhalten.

Das Ausgangsmaterial wurde wie folgt hergestellt:

Aus Diäthyl-1-naphthylmalonat und Formamidinacetat wurde das 5-(1-Naphthylmethyl)-4,6(1H,5H)-pyrimidindion, Schmelzpunkt >270°C, und daraus nach Trocknen unter vermindertem Druck bei 80°C durch Umsetzung mit POCl₃ das 4,6-Dichlor-5-(1-naphthylmethyl)pyrimidin, Schmelzpunkt 111-112°C (aus Methylenchlorid und Isopropyläther) hergestellt.

Umsetzung dieser Verbindung mit p-Chlorphenylsulfonamid lieferte p-Chlor-N--[6-chlor-5-(1-naphthylmethyl)-4-pyrimidinyl]benzolsulfonamid, Schmelzpunkt 202-203°C (aus Acetonitril).

### Beispiel 101

In Analogie zu Beispiel 1 wurde aus N-[6-Chlor-5-(p-isopropylphenyl)-4-pyrimidinyl]-p-isopropylbenzolsulfonamid und Aethylenglykol-Na das N-6-(2-Hydroxyäthoxy)-5-(p-isopropylphenyl)-4-pyrimidinyl]-p-isopropylbenzolsulfonamid, Schmelzpunkt 142-144°C (aus Isopropyläther) erhalten.

Das Ausgangsmaterial wurde wie folgt hergestellt:

Aus Diäthyl-(p-isopropylphenyl)malonat und Formamidinacetat wurde das 5-(p-Isopropylphenyl)-4,6(1H,5H)-pyrimidindion, Schmelzpunkt >290°C, und daraus durch Umsetzung mit POCl₃ das 4,6-Dichlor-5-(p-isopropylphenyl)pyrimidin, Schmelzpunkt 69-70°C (aus n-Hexan) erhalten. Umsetzung dieser Verbindung mit p-Isopropylbenzolsulfonamid lieferte N-[6-Chlor-5-(p-isopropylphenyl)-4-pyrimidinyl]-p-isopropylbenzolsulfonamid, Schmelzpunkt 198-199°C (aus Acetonitril und Isopropyläther).

### Beispiel 102

In Analogie zu Beispiel 1 wurde aus N-[6-Chlor-5-(p-isopropylphenyl)-4-pyrimidinyl]-p-cyclopentylbenzolsulfonamid und Aethylenglykol-Na das p-Cyclopentyl-N-[6-(2-hydroxyäthoxy)-5-(p-isopropylphenyl)-4-pyrimidinyl]benzolsulfonamid, Schmelzpunkt 132°C (Zersetzung) (aus Aceton-Isopropyläther) erhalten.

Das Ausgangsmaterial wurde aus 4,6-Dichlor-5-(p-isopropylphenyl)pyrimidin und p-Cyclopentylbenzolsulfonamid hergestellt, Schmelzpunkt 188-189°C (aus Methylenchlorid und Isopropyläther).

### Beispiel 103

Aus p-t-Butyl-N-[6-chlor-5-(o-methoxybenzyl)-4-pyrimidinyl]benzolsulfonamid und Pyridin-2-carbonsäure wird durch Erhitzen in Methylenchlorid in Gegenwart von Dicydohexylcarbodiimid das 4-t-Butyl-N-[6-[2-(pyridin-2-ylcarbonyloxy)äthoxy]-5-(2-methoxybenzyl)pyrimidin-4-yl]benzolsulfonamid erhalten.

Analog können erhalten werden 4-t-Butyl-N-[6-[2-(pyridin-3-ylcarbonyloxy)äthoxy]-5-(2-methoxybenzyl)pyrimidin-4-yl]benzolsulfonamid; 4-t-Butyl-N-[6-[2-(pyridin-4-ylcarbonyloxy)äthoxy]-5-(2-methoxybenzyl)pyrimidin-4-yl]benzolsulfonamid; 4-t-Butyl-N-[6-[2-[(3-methylisoxazol-5-yl)carbonyloxy]äthoxy]-5-(2-methoxybenzyl)pyrimidin-4-yl]benzolsulfonamid; 4-t-Butyl-N-[6-[2-(furan-2-ylcarbonyloxy)äthoxy]-5-(2-methoxybenzyl)pyrimidin-4-yl]benzolsulfonamid; 4-t-Butyl-N-[6-[2-(furan-3-ylcarbonyloxy)äthoxy]-5-(2-methoxybenzyl)pyrimidin-4-yl]benzolsulfonamid; 4-t-Butyl-N-[6-[2-(thiophen-2-yl-carbonyl)äthoxy]-5-(2-methoxybenzyl)pyrimidin-4-yl]benzolsulfonamid; 4-t-Butyl-N-[6-[2-(thiophen-3-ylcarbonyl)äthoxy]-5-(2-methoxybenzyl)pyrimidin-4-yl]benzolsulfonamid.

### Beispiel 104

Aus Diäthyl p-tolylmalonat und 2-Pyrimidincarboximidamid erhält man das (R,S)-5-(4-Methyl-phenyl)-2-(pyrimidin-2-yl)-1,4,5,6-tetrahydro-pyrimidin-4,6-dion und daraus mit POCl₃ das 4,6-Dichlor-5-(4-methyl-phenyl)-2,2'-bipyrimidinyl, Schmelzpunkt 214-215°C (aus Toluol-Hexan).

Umsetzung dieser Verbindung mit 4-tert.Butylphenylsulfonamid-Kalium liefert 4-tert-Butyl-N-[6-chlor-5-(4-methyl-phenyl)-2,2'-bipyrimidin-4-yl]-benzolsulfonamid-Kalium, aus dem mit Natriumglykolat das 4-tert-Butyl-N-[6-(2-hydroxy-äthoxy)-5-(4-methyl-phenyl)-2,2'-dipyrimidin-4-yl]-benzolsulfonamid erhalten wird.

### Beispiel A

Tabletten enthaltend die folgenden Bestandteile können in herkömmlicher Weise hergestellt werden:

| Bestandteile | pro Tablette |
|---|---|
| Verbindung der Formel I | 10,0 - 100,0 mg |
| Lactose | 125,0 mg |
| Maisstärke | 75,0 mg |
| Talk | 4,0 mg |
| Magnesiumstearat und Isopropyläther). | 1,0 mg |

### Beispiel B

Kapseln enthaltend die folgenden Bestandteile können in herkömmlicher Weise hergestellt werden:

| Bestandteile | pro Kapsel |
|---|---|
| Verbindung der Formel I | 25,0 mg |
| Lactose | 150,0 mg |
| Maisstärke | 20,0 mg |
| Talk | 5,0 mg |

### Beispiel C

Injektionslösungen können folgende Zusammensetzung aufweisen:

| | |
|---|---|
| Verbindung der Formel I | 3,0 mg |
| Gelatine | 150,0 mg |
| Phenol | 4,7 mg |
| Wasser für Injektionslösungen | ad 1,0 ml |

### Beispiel D

In 3,5 ml Myglyol 812 und 0,08 g Benzylalkohol werden 500 mg Verbindung der Formel I suspendiert. Diese Suspension wird in einen Behälter mit Dosierventil eingefüllt. Es werden 5,0 g Freon 12 unter Druck durch das Ventil in den Behälter abgefüllt. Durch Schütteln wird das Freon in der Myglyol-Benzylalkoholmischung gelöst. Dieser Spraybehälter enthält ca. 100 Einzeldosen, die einzeln appliziert werden können.

## Patentansprüche

1. Verwendung von Verbindungen der Formel 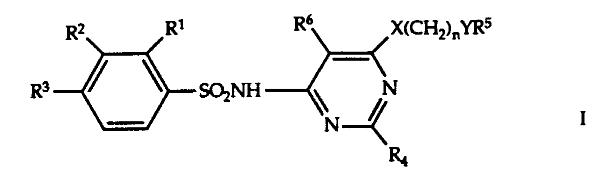 worin
R¹ Wasserstoff, nieder-Alkyl, nieder-Alkoxy, nieder-Alkylthio, Halogen oder Trifluormethyl;
R² Wasserstoff, Halogen, nieder-Alkoxy, Hydroxy-nieder-alkoxy oder Trifluormethyl;
R³ Wasserstoff, Hydroxy, Halogen, Alkylthio, Cycloalkyl, Hydroxynieder-alkyl, Hydroxy-nieder-alkoxy, Hydroximino-nieder-alkyl, nieder-Alkenyl, Oxo-nieder-alkyl, Trifluormethyl, Trifluormethoxy, nieder-Alkoxy, nieder-Alkoxy-nieder-alkoxy oder Aryl-nieder-alkoxy;
R² und R³ zusammen Butadienyl;
R⁴ Wasserstoff, nieder-Alkyl, Aryl oder Heteroaryl;
R⁵ Wasserstoff, nieder-Alkanoyl, Benzoyl, Heterocyclyl-carbonyl oder Tetrahydropyran-2-yl;
R⁶ einen Rest der Formel 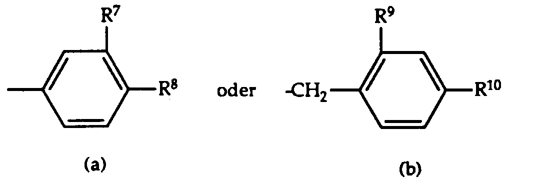
R⁷ Wasserstoff, nieder-Alkoxy oder Nitro;
R⁸ Wasserstoff, Halogen, nieder-Alkyl, nieder-Alkoxy, nieder-Alkylthio, Nitro, Hydroxy, Amino oder Trifluormethyl;
R⁷ und R⁸ zusammen Butadienyl;
R⁹ Wasserstoff, Halogen, nieder-Alkyl, nieder-Alkoxy, nieder-Alkylthio, oder Trifluormethyl;
R¹⁰ Wasserstoff, Halogen, nieder-Alkyl, nieder-Alkoxy oder nieder-Alkylthio;
X und Y unabhängig voneinander O, S oder NH; und
n 2,3 oder 4 bedeuten; wobei "nieder" Reste mit 1-7C-Atomen bezeichnet,
und Salzen davon als Wirkstoffe bei der Herstellung von Heilmitteln zur Behandlung von Kreislauferkrankungen, insbesondere Hypertonie, Ischämie, Vasospasmen und Angina pectoris.

2. Verwendung gemäss Anspruch 1 von Verbindungen der Formel I, und Salzen davon, in denen R⁴ Wasserstoff, nieder-Alkyl oder Aryl ist und die übrigen Symbole die in Anspruch 1 angegebene Bedeutung haben.

3. Verwendung gemäss Anspruch 1 von Verbindungen der Formel I von Anspruch 1 und Salze davon, worin R⁶ einen Rest der Formel 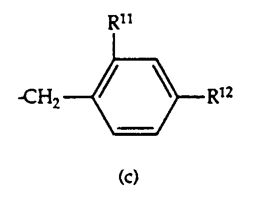 darstellt, und R¹¹ Halogen, nieder-Alkoxy, nieder-Alkylthio oder Trifluormethyl; und R¹² Wasserstoff oder nieder-Alkoxy bedeuten und R¹-R⁵, X, Y und n die in Anspruch 1 angegebene Bedeutung haben.

4. Verwendung gemäss Anspruch 1 von Verbindungen der Formel I und Salze davon, in denen R⁴ Wasserstoff, nieder-Alkyl oder Aryl ist und die übrigen Symbole die in Anspruch 3 angegebene Bedeutung haben.

5. Verwendung gemäss Anspruch 1 der Verbindungen
N-[5-(2,6-Dimethoxybenzyl)-6-(3-hydroxypropyl)-4-pyrimidinyl]-p-vinylbenzolsulfonamid,
α,α,α-Trifluor-N-[6-(2-hydroxyäthoxy)-6-[o-(trifluormethyl)benzyl]pyrimidinyl]-p-toluolsulfonamid,
N-[6-(2-Hydroxyäthoxy)-5-[o(trifluormethyl)benzyl]-4-pyrimidinyl]-p-methoxybenzolsulfonamid,
p-Chlor-N-[6-(2-hydroxyäthoxy)-5-[o-(trifluormethyl)benzyl]-4-pyrimidinyl]benzolsulfonamid,
N-[6-(2-Hydroxyäthoxy)-5-(o-methoxybenzyl)-4-pyrimidinyl]-p-vinylbenzolsulfonamid,
N-[6-(2-Hydroxyäthoxy)-5-[o-(trifluormethyl)benzyl]-4-pyrimidinyl]-p-(methylthio)benzolsulfonamid,
N-[5-(2,4-Dimethoxybenzyl)-6-(2-hydroxyäthoxy)-4-pyrimidinyl]-p-isopropylbenzolsulfonamid,
rac-N-[5-(o-Methoxybenzyl)-6-[2-[(tetrahydro-2H-pyran-2-yl)oxy]äthoxy]-4-pyrimidinyl]-p-vinylbenzolsulfonamid,
rac-p-[[5-(o-Methoxybenzyl)-6-[2-[(tetrahydro-2H-pyran-2-yl)oxy]äthoxy]-4-pyrimidinyl]sulfamoyl]benzaldehyd,
p-[(RS)-1-Hydroxyäthyl]-N-[5-(-methoxybenzyl)-6-[2-[[(RS)-tetrahydro-2H-pyran-2-yl]oxy]äthoxy]-4-pyrimidinyl]benzolsulfonamid,
rac-α-Hydroxy-N-[5-(o-methoxybenzyl)-6[2-[(tetrahydro-2H-pyran-2-yl)oxy]äthoxy]-4-pyrimidinyl]-p-toluolsulfonamid,
rac-α-[(>E/Z)-Hydroxyimino-N-[5-(o-methoxybenzyl)-6-[2-[(tetrahydro-2H-pyran-2-yl)oxy]äthoxy]-4-pyrimidinyl]-p-toluolsulfonamid,
rac-N-6-(2-Hydroxyäthoxy)-5-(o-methoxybenzyl)-p-(1-hydroxyäthyl)benzolsulfonamid,
α-[(E/Z)-Hydroxyimino]-N-[6-(2-hydroxyäthoxy)-5-(o-methoxybenzyl)-4-pyrimidinyl]-p-toluolsulfonamid,
p-[[6-(2-Hydroxyäthoxy)-5-(o-methoxybenzyl)-4-pyrimidinyl]sulfamoyl]benzaldehyd,
2-[[5-(o-Methoxybenzyl)-6-[(p-vinylphenyl)sulfamoyl]-4-pyrimidinyl]oxy]äthylacetat,
N--[6(2-Hydroxyäthoxy)-5-(o-methoxybenzyl)-4-pyrimidinyl]-p-methoxybenzolsulfonamid,
N--[6-(2-Hydroxyäthoxy)-5-(o-chlorbenzyl)-4-pyrimidinyl]-p-toluolsulfonamid,
N-[6-(2-Hydroxyäthoxy)-5-(o-methoxybenzyl)-4-pyrimidinyl]-p-methylthiobenzolsulfonamid,
N--[6-(2-Hydroxyäthoxy)-5-(o-methoxybenzyl)-4-pyrimidinyl]-α,α,α-trifluor-p-toluolsulfonamid,
N-[6-(2-Hydroxyäthoxy)-5-(o-methoxybenzyl)-4-pyrimidinyl]-p-isopropylbenzolsulfonamid,
p-t-Butyl-N-[6-(2-hydroxyäthoxy)-5-(o-methoxybenzyl)4-pyrimidinyl]benzolsulfonamid,
N-[6-(2-Hydroxyäthoxy)-5-(o-chlorbenzyl)-4-pyrimidinyl]-p-isopropylbenzolsulfonamid,
N--[6-(2-Hydroxyäthoxy)-5-(o-methylthiobenzyl)-4-pyrimidinyl]-p-isopropylbenzolsulfonamid,
N-[6-(2-Hydroxyäthoxy)-5-(o-chlorbenzyl)-4-pyrimidinyl]-p-isobutylbenzolsulfonamid,
N-[6-(2-Hydroxyäthoxy)-5-(o-chlorbenzyl)-4-pyrimidinyl]-p-cyclohexylbenzolsulfonamid,
N-[6-(2-Hydroxyäthoxy)-5-(o-chlorbenzyl)-4-pyrimidinyl]-p-isopentylbenzolsulfonamid,
N--[6(2-Hydroxyäthoxy)-5(o-methoxybenzyl)-4-pyrimidinyl]-p-isopropylthiobenzolsulfonamid.

6. Verwendung gemäss Anspruch 1 von Verbindung der Formel I von Anspruch 1, und Salzen davon worin R⁶ einen Rest der Formel 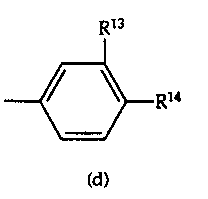 darstellt, und R¹³ Wasserstoff, nieder-Alkoxy oder Nitro; und R¹⁴ Wasserstoff, Halogen, nieder-Alkyl; nieder-Alkoxy, nieder-Alkylthio oder Nitro bedeuten, oder R¹³ und R¹⁴ gemeinsam Butadienyl bedeuten, und R¹-R⁵, X, Y und n die in Anspruch 1 angegebene Bedeutung haben.

7. Verwendung gemäss Anspruch 1 von Verbindungen der Formel I und Salze davon, in denen R⁴ Wasserstoff, nieder-Alkyl oder Aryl ist und die übrigen Symbole die in Anspruch 6 angegebene Bedeutung haben.

8. Verwendung gemäss Anspruch 1 der Verbindungen
N-[5(p-Chlorphenyl)-6-(2-hydroxyäthoxy)-4-pyrimidinyl]-α,α,α,-trifluor-p-toluolsulfonamid,
N-[5(p-Chlorphenyl)-6-(2-hydroxyäthoxy)-4-pyrimidinyl]-p-(trifluormethoxy)benzolsulfonamid,
p-Chlor-N-[5-(m-chlorphenyl)-6-(2-hydroxyäthoxy)-4-pyrimidinyl]benzolsulfonamid,
p-Chlor-N-[5-(p-fluorphenyl)-6-(2-hydroxyäthoxy)-4-pyrimidinyl]benzolsulfonamid,
N-[5(p-Chlorphenyl)-6-(2-hydroxyäthoxy)-4-pyrimidinyl]-p-fluorbenzolsulfonamid,
o-Chlor-N-[5-(p-chlorphenyl)-6-(2-hydroxyäthoxy)-4-pyrimidinyl]benzolsulfonamid,
p-Chlor-N-[5-(3,4-dimethoxyphenyl)-6-(2-hydroxyäthoxy)-4-pyrimidinyl]benzolsulfonamid,
3,4-Dichlor-N-[5-(p-chlorphenyl)-6-(2-hydroxyäthoxy)-4-pyrimidinyl]benzolsulfonamid,
N-5-(p-Chlorphenyl)-6-(2-hydroxyäthoxy)-4-pyrimidinyl]-α,α,α,α',α',α'-hexafluor-3,5-xylolsulfonamid,
3-Chlor-N-[5-(p-chlorphenyl)-6-(2-hydroxyäthoxy)-4-pyrimidinyl]-4-(2-hydroxyäthoxy)benzolsulfonamid,
p-Chlor-N-(6-(2-hydroxyäthoxy)-5-(p-nitrophenyl)-4-pyrimidinyl]benzolsulfonamid,
p-Butoxy-N-[5-(p-chlorphenyl)-6-(2-hydroxyäthoxy)-4-pyrimidinyl]benzolsulfonamid,
N-[5-(p-Chlorphenyl)-6-(2-hydroxyäthoxy)-4-pyrimidinyl]-3,4-dimethoxybenzolsulfonamid,
2-Chlor-N-[5-(p-chlorphenyl)-6-(2-hydroxyäthoxy)-4-pyrimidinyl]-α,α,α-trifluor-p-toluolsulfonamid,
6-Chlor-N-[5-(p-chlorphenyl)-6-(2-hydroxyäthoxy)-4-pyrimidinyl]-α,α,α-trifluor-m-toluolsulfonamid,
2,3,4-Trichlor-N-[(5-chlorphenyl)-6-(2-hydroxyäthoxy)-4-pyrimidinyl]benzolsulfonamid,
m-Chlor-N-(p-chlorphenyl)-6-(2-hydroxyäthoxy)-4-pyrimidinyl]benzolsulfonamid,
2,4-Dichlor-N-[5-(p-chlorphenyl)-6-(2-hydroxyäthoxy)-4-pyrimidinyl]benzolsulfonamid,
N-5-(p-Chlorphenyl)-6-(2-hydroxyäthoxy)-α,α,α-trifluor-m-toluolsulfonamid,
N-5-(p-Chlorphenyl)-6-(2-hydroxyäthoxy)-4-pyrimidinyl]-α,α,α-trifluor-o-toluolsulfonamid,
N-[5-(p-Chlorphenyl)-6-(2-hydroxyäthoxy)-4-pyrimidinyl]-2-naphthalinsulfonamid,
p-Chlor-N-[6-(2-hydroxyäthoxy)-5-(m-nitrophenyl)-4-pyrimidinyl]benzolsulfonamid,
α,α,α-Trifluor-N-[6-(2-hydroxyäthoxy)-5-(m-nitrophenyl)4-pyrimidinyl]-p-toluolsulfonamid,
p-(Benzyloxy)-N--[5-(p-chlorphenyl)-6-(2-hydwxyäthoxy)4-pyrimidinyl]benzolsulfonamid,
N-[5-(p-Chlorphenyl)-4-pyrimidinyl]-p-hydroxybenzolsulfonamid,
N-[5-(p-Chlorphenyl)-6-(2-hydroxyäthoxy)-4-pyrimidinyl]-p-(2-methoxyäthoxy)benzolsulfonamid,
N-[5-(p-Bromphenyl)-6-(2-hydroxyäthoxy)-4-pyrimidinyl]-p-chlorbenzolsulfonamid,
p-Chlor-N-[6-(2-hydroxyäthoxy)-5-p-tolyl-4-pyrimidinyl]benzolsulfonamid,
N-[5(p-Chlorphenyl)-6-(2-hydroxyäthoxy)-4-pyrimidinyl]-α,α,α-trffluor-p-toluolsulfonamid-Natriumsalz,
N-[6-(2-Hydroxyäthoxy)-5-(p-methoxyphenyl)-4-pyrimidinyl]-p-toluolsulfonamid,
N-[6-(2-Hydroxyäthoxy)-5-(p-methoxyphenyl)-4-pyrimidinyl]-p-methoxybenzolsulfonamid,
N-[6-(2-Hydroxyäthoxy)-5-(p-methoxyphenyl)-4-pyrimidinyl]-p-(methylthio)benzolsulfonamid,
N-[6-(2-Hydroxyäthoxy)-5-(p-methoxyphenyl)-2-methyl-4-pyrimidinyl]-p-methoxybenzolsulfonamid,
N-[6-(2-Hydroxyäthoxy)-5-(p-methoxyphenyl)-4-pyrimidinyl]-p-isopropylbenzolsulfonamid,
p-t-Butyl-N-[6-(2-hydroxyäthoxy)-5-(p-methoxyphenyl)-4-pyrimidinyl]benzolsulfonamid,
rac-p-sek-Butyl-N-[6-(2-hydroxyäthoxy)-5-(p-methoxyphenyl)-4-pyrimidinyl]benzolsulfonamid,
N-[6-(2-Hydroxyäthoxy)-5-[p-(methylthio)phenyl]-4-pyrimidinyl]-p-isopropylbenzolsulfonamid,
N-[6-(2-Hydroxyäthoxy)-5-[p-(methylthio)phenyl]-4-pyrimidinyl]-α,α,α-trifluor-p-toluolsulfonamid,
p-Chlor-N-[5-(p-chlorphenyl)-6-(2-hydroxyäthoxy)-2-methyl-4-pyrimidinyl]benzolsulfonamid,
p-Chlor-N-[6-(2-hydroxyäthoxy)-5-(p-nitrophenyl)-4-pyrimidinyl]benzolsulfonamid,
N-[5(p-Aminophenyl)-6-(2-hydroxyäthoxy)-4-pyrimidinyl]-p-chlorbenzolsulfonamid-hydrochlorid,
N-[5-(4-Biphenylyl)-6-(2-hydroxyäthoxy)-4-pyrimidinyl]-p-chlorbenzolsulfonamid,
p-Chlor-N-[(6-hydroxyäthoxy)-5-(α,α,α-trifluor-p-tolyl)-4-pyrimidinyl]benzolsulfonamid,
p-Chlor-N-[5-(p-hydroxyphenyl)-6-(2-hydroxyäthoxy)-4-pyrimidinyl]benzolsulfonamid,
N-[5-[p-Benzyloxy)phenyl]-6-(2-hydroxyäthoxy)-4-pyrimidinyl]-p-chlorbenzolsulfonamid,
N-[6-(2-Hydroxyäthoxy)-5-(α,α,α-trifluor-p-tolyl)-4-pyrimidinyl]-α,α,α-trifluor-p-toluolsulfonamid,
N-[5-(p-Chlorphenyl)-6-(2-hydroxyäthoxy)-4-pyrimidinyl]-p-isopropylbenzolsulfonamid,
N--[6-Hydroxyäthoxy)-5-p-tolyl-4-pyrimidinyl]-p-isopropylbenzolsulfonamid,
p-tert-Butyl-N-[6-(2-hydroxyäthoxy)-5-p-tolyl-4-pyrimidinyl]benzolsulfonamid,
N-[6-(2-Hydroxyäthoxy)-5-p-tolyl-4-pyrimidinyl]-p-(2-methoxyäthoxy)benzolsulfonamid,
N-[6-(2-Hydroxyäthoxy)-5-p-tolyl-4-pyrimidinyl]-p-(trifluormethoxy)benzolsulfonamid,
p-Butyl-N-[6-(2-hydroxyäthoxy)-5-p-tolyl-4-pyrimidinyl]benzolsulfonamid,
N-[6-(2-Hydroxyäthoxy)-5-p-tolyl-4-pyrimidinyl]-2-naphthalinsulfonamid,
N-[6-(2-Hydroxyäthoxy)-5-p-tolyl-4-pyrimidinyl]-p-toluolsulfonamid,
N-[6-(2-Hydroxyäthoxy)-5-p-tolyl-4-pyrimidinyl]-α,α,α-trifluor-p-toluolsulfonamid,
p-(2-Hydroxyäthoxy)-N-[6-(2-hydroxyäthoxy)-5-p-tolyl-4-pyrimidinyl]benzolsulfonamid,
N-[6-(2-Hydroxyäthoxy)-5-p-tolyl-4-pyrimidinyl]-p-propylbenzolsulfonamid,
N-[6-(2-Hydroxyäthoxy)-5-p-tolyl-4-pyrimidinyl]-o-propylbenzolsulfonamid,
p-Aethyl-N-[6-(2-hydroxyäthoxy)-5-p-tolyl-4-pyrimidinyl]benzolsulfonamid,
o-Aethyl-N-[6-(2-hydroxyäthoxy)-5-p-tolyl-4-pyrimidinyl]benzolsulfonamid,
p-Cyclopentyl-N-[6-(2-hydroxyäthoxy)-5-p-tolyl]benzolsulfonamid,
α,α,α-Trifluor-N-[6-(2-hydroxyäthoxy)-5-p-tolyl-4-pyrimidinyl]-o-toluolsulfonamid,
N-[6(2-Hydroxyäthoxy)-5-p-tolyl-4-pyrimidinyl]-o-toluolsulfonamid,
p-Chlor-N-[6-(2-hydroxyäthoxy)-5-(1-naphthylmethyl)-4-pyrimidinyl]benzolsulfonamid,
N-6-(2-Hydroxyäthoxy)-5-(p-isopropylphenyl)-4-pyrimidinyl]-p-isopropylbenzolsulfonamid,
p-Cyclopentyl-N-[6-(2-hydroxyäthoxy)-5-(p-isopropylphenyl)-4-pyrimidinyl]benzolsulfonamid,
N-[6-(2-Hydroxyäthoxy)-5-(α,α,α-trifluor-p-tolyl)-4-pyrimidinyl]-p-isopropylbenzolsulfonamid,
N-[5-(p-Bromphenyl)-6-(2-hydroxyäthoxy)-4-pyrimidinyl]-p-isopropylbenzolsulfonamid,
N-[6(2-Hydroxyäthoxy)-5-(p-äthylphenyl)-4-pyrimidinyl]-pisopropyl]benzolsulfonamid,
p-Cyclopentyl-N-[6(2-hydroxyäthoxy)-5-(p-äthylphenyl)-4-pyrimidinyl]benzolsulfonamid.

## Claims

1. The use of compounds of the formula 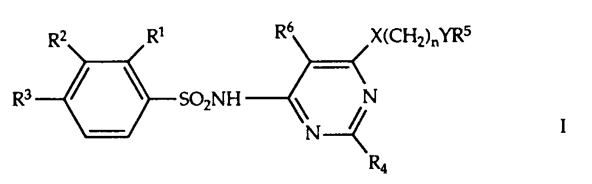 wherein
R¹ signifies hydrogen, lower-alkyl, lower-alkoxy, lower-alkylthio, halogen or trifluoromethyl;
R² signifies hydrogen, halogen, lower-alkoxy, hydroxy-lower-alkoxy or trifluoromethyl; and
R³ signifies hydrogen, hydroxy, halogen, alkylthio, cycloalkyl, hydroxy-lower-alkyl, hydroxy-lower-alkoxy, hydroximino-lower-alkyl, lower-alkenyl, oxo-lower-alkyl, trifluoromethyl, trifluoromethoxy, lower-alkoxy, lower-alkoxy-lower-alkoxy or aryl-lower-alkoxy;
R² and R³ together signify butadienyl;
R⁴ signifies hydrogen, lower-alkyl, aryl or heteroaryl;
R⁵ signifies hydrogen, lower-alkanoyl, benzoyl, heterocyclyl-carbonyl or tetrahydropyran-2-yl;
R⁶ signifies a residue of the formula 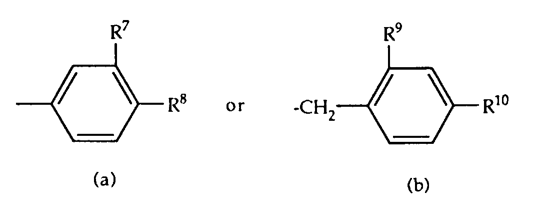
R⁷ signifies hydrogen, lower-alkoxy or nitro; and
R⁸ signifies hydrogen, halogen, lower-alkyl, lower-alkoxy, lower-alkylthio, nitro, hydroxy, amino or trifluoromethyl;
R⁷ and R⁸ together signify butadienyl;
R⁹ signifies hydrogen, halogen, lower-alkyl, lower-alkoxy, lower-alkylthio, or trifluoromethyl;
R¹⁰ signifies hydrogen, halogen, lower-alkyl, lower-alkoxy or lower-alkylthio;
X and Y each independently signify O, S or NH; and
n signifies 2, 3 or 4; whereby "lower" denotes residues with 1-7 C atoms;
and salts thereof as active ingredients for the manufacture of medicaments for the treatment of circulatory disorders, especially hypertension, ischemia, vasospasms and angina pectoris.

2. The use according to claim 1 of compounds of formula I and salts thereof in which R⁴ is hydrogen, lower-alkyl or aryl and the remaining symbols have the significance given in claim 1.

3. The use according to claim 1 of compounds of formula I of claim 1 and salts thereof, wherein R⁶ represents a residue of the formula 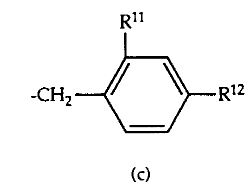 and R¹¹ signifies halogen, lower-alkoxy, lower-alkylthio or trifluoromethyl; and R¹² signifies hydrogen or lower-alkoxy and R¹-R⁵, X and Y and n have the significance given in claim 1.

4. The use according to claim 1 of compounds of formula I and salts thereof in which R⁴ is hydrogen, lower-alkyl or aryl and the remaining symbols have the significance given in claim 3.

5. The use according to claim 1 of the compounds
N-[5-(2,6-dimethoxybenzyl)-6-(3-hydroxypropyl)-4-pyrimidinyl]-p-vinylbenzenesulphonamide,
α,α,α-trifluoro-N-[6-(2-hydroxyethoxy)-6-[o-(trifluoromethyl)benzyl]pyrimidinyl]-p-toluenesulphonamide,
N-[6-(2-hydroxyethoxy)-5-[o-(trifluoromethyl)benzyl]-4-pyrimidinyl]-p-methoxybenzenesulphonamide,
p-chloro-N-[6-(2-hydroxyethoxy)-5-[o-(trifluoromethyl)benzyl]-4-pyrimidinyl]benzenesulphonamide,
N-[6-(2-hydroxyethoxy)-5-(o-methoxybenzyl)-4-pyrimidinyl]-p-vinylbenzenesulphonamide,
N-[6-(2-hydroxyethoxy)-5-[o-(trifluoromethyl)benzyl]-4-pyrimidinyl]-p-(methylthio)benzenesulphonamide,
N-[5-(2,4-dimethoxybenzyl)-6-(2-hydroxyethoxy)-4-pyrimidinyl]-p-isopropylbenzenesulphonamide,
rac-N-[5-(o-methoxybenzyl)-6-[2-[(tetrahydro-2H-pyran-2-yl)oxy]ethoxy]-4-pyrimidinyl]-p-vinylbenzenesulphonamide,
rac-p-[[5-(o-methoxybenzyl)-6-[2-[(tetrahydro-2H-pyran-2-yl)oxy]ethoxy]-4-pyrimidinyl]sulphamoyl]benzaldehyde,
p-[(RS)-1-hydroxyethyl]-N-[5-(-methoxybenzyl)-6-[2-[[(RS)-tetrahydro-2H-pyran-2-yl]oxy]ethoxy]-4-pyrimidinyl]benzenesulphonamide,
rac-α-hydroxy-N-[5-(o-methoxybenzyl)-6-[2-[(tetrahydro-2H-pyran-2-yl)oxy]ethoxy]-4-pyrimidinyl]-p-toluenesulphonamide,
rac-α-[(>E/Z)-hydroxyimino-N-[5-(o-methoxybenzyl)-6-[2-[(tetrahydro-2H-pyran-2-yl)oxy]ethoxy]-4-pyrimidinyl]-p-toluenesulphonamide,
rac-N-6-(2-hydroxyethoxy)-5-(o-methoxybenzyl)-p-(1-hydroxyethyl)benzenesulphonamide,
α-[(E/Z)-hydroxyimino]-N-[6-(2-hydroxyethoxy)-5-(o-methoxybenzyl)-4-pyrimidinyl]-p-toluenesulphonamide,
p-[[6-(2-hydroxyethoxy)-5-(o-methoxybenzyl)-4-pyrimidinyl]sulphamoyl]benzaldehyde,
2-[[5-(o-methoxybenzyl)-6-[(p-vinylphenyl)sulphamoyl]-4-pyrimidinyl]oxy]ethyl acetate,
N-[6-(2-hydroxyethoxy)-5-(o-methoxybenzyl)-4-pyrimidinyl]-p-methoxybenzenesulphonamide,
N-[6-(2-hydroxyethoxy)-5-(o-chlorobenzyl)-4-pyrimidinyl]-p-toluenesulphonamide,
N-[6-(2-hydroxyethoxy)-5-(o-methoxybenzyl)-4-pyrimidinyl]-p-methylthiobenzenesulphonamide,
N-[6-(2-hydroxyethoxy)-5-(o-methoxybenzyl)-4-pyrimidinyl]-α,α,α-trifluoro-p-toluenesulphonamide,
N-[6-(2-hydroxyethoxy)-5-(o-methoxybenzyl)-4-pyrimidinyl]-p-isopropylbenzenesulphonamide,
p-t-butyl-N-[6-(2-hydroxyethoxy)-5-(o-methoxybenzyl)-4-pyrimidinyl]benzenesulphonamide,
N-[6-(2-hydroxyethoxy)-5-(o-chlorobenzyl)-4-pyrimidinyl]-p-isopropylbenzenesulphonamide,
N-[6-(2-hydroxyethoxy)-5-(o-methylthiobenzyl)-4-pyrimidinyl]-p-isopropylbenzenesulphonamide,
N-[6-(2-hydroxyethoxy)-5-(o-chlorobenzyl)-4-pyrimidinyl]-p-isobutylbenzenesulphonamide,
N-[6-(2-hydroxyethoxy)-5-(o-chlorobenzyl)-4-pyrimidinyl]-p-cyclohexylbenzenesulphonamide,
N-[6-(2-hydroxyethoxy)-5-(o-chlorobenzyl)-4-pyrimidinyl]-p-isopentylbenzenesulphonamide,
N-[6-(2-hydroxyethoxy)-5-(o-methoxybenzyl)-4-pyrimidinyl]-p-isopropylthiobenzenesulphonamide.

6. The use according to claim 1 of compounds of formula I of claim 1 and salts thereof, wherein R⁶ represents a residue of the formula 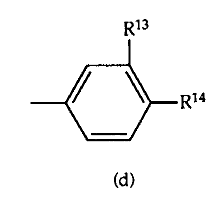 and R¹³ signifies hydrogen, lower-alkoxy or nitro; and R¹⁴ signifies hydrogen, halogen, lower-alkyl; lower-alkoxy, lower-alkylthio or nitro or R¹³ and R¹⁴ together signify butadienyl and R¹-R⁵, X, Y and n have the significance given in claim 1.

7. The use according to claim 1 of compounds of formula I and salts thereof in which R⁴ is hydrogen, lower-alkyl or aryl and the remaining symbols have the significance given in claim 6.

8. The use according to claim 1 of the compounds
N-[5-(p-chlorophenyl)-6-(2-hydroxyethoxy)-4-pyrimidinyl]-α,α,α-trifluoro-p-toluenesulphonamide,
N-[5-(p-chlorophenyl)-6-(2-hydroxyethoxy)-4-pyrimidinyl]-p-(trifluoromethoxy)benzenesulphonamide,
p-chloro-N-[5-(m-chlorophenyl)-6-(2-hydroxyethoxy)-4-pyrimidinyl]benzenesulphonamide,
p-chloro-N-[5-(p-fluorophenyl)-6-(2-hydroxyethoxy)-4-pyrimidinyl]benzenesulphonamide,
N-[5-(p-chlorophenyl)-6-(2-hydroxyethoxy)-4-pyrimidinyl]-p-fluorobenzenesulphonamide,
o-chloro-N-[5-(p-chlorophenyl)-6-(2-hydroxyethoxy)-4-pyrimidinyl]benzenesulphonamide,
p-chloro-N-[5-(3,4-dimethoxyphenyl)-6-(2-hydroxyethoxy)-4-pyrimidinyl]benzenesulphonamide,
3,4-dichloro-N-[5-(p-chlorophenyl)-6-(2-hydroxyethoxy)-4-pyrimidinyl]benzenesulphonamide,
N-5-(p-chlorophenyl)-6-(2-hydroxyethoxy)-4-pyrimidinyl]-α,α,α-α',α',α'-hexafluoro-3,5-xylenesulphonamide,
3-chloro-N-[5-(p-chlorophenyl)-6-(2-hydroxyethoxy)-4-pyrimidinyl]-4-(2-hydroxyethoxy)benzenesulphonamide,
p-chloro-N-(6-(2-hydroxyethoxy)-5-(p-nitrophenyl)-4-pyrimidinyl]benzenesulphonamide,
p-butoxy-N-[5-(p-chlorophenyl)-6-(2-hydroxyethoxy)-4-pyrimidinyl]benzenesulphonamide,
N-[5-(p-chlorophenyl)-6-(2-hydroxyethoxy)-4-pyrimidinyl]-3,4-dimethoxybenzenesulphonamide,
2-chloro-N-[5-(p-chlorophenyl)-6-(2-hydroxyethoxy)-4-pyrimidinyl]-α,α,α-trifluoro-p-toluenesulphonamide,
6-chloro-N-[5-(p-chlorophenyl)-6-(2-hydroxyethoxy)-4-pyrimidinyl]-a,a,a-trifluoro-m-toluenesulphonamide,
2,3,4-trichloro-N-[(5-chlorophenyl)-6-(2-hydroxyethoxy)-4-pyrimidinyl]benzenesulphonamide,
m-chloro-N-(p-chlorophenyl)-6-(2-hydroxyethoxy)-4-pyrimidinyl]benzenesulphonamide,
2,4-dichloro-N-[5-(p-chlorophenyl)-6-(2-hydroxyethoxy)-4-pyrimidinyl]benzenesulphonamide,
N-5-(p-chlorophenyl)-6-(2-hydroxyethoxy)-α,α,α-trifluoro-m-toluenesulphonamide,
N-5-(p-chlorophenyl)-6-(2-hydroxyethoxy)-4-pyrimidinyl]-α,α,α-trifluoro-o-toluenesulphonamide,
N-[5-(p-chlorophenyl)-6-(2-hydroxyethoxy)-4-pyrimidinyl]-2-naphthalenesulphonamide,
p-chloro-N-[6-(2-hydroxyethoxy)-5-(m-nitrophenyl)-4-pyrimidinyl]benzenesulphonamide,
α,α,α-trifluoro-N-[6-(2-hydroxyethoxy)-5-(m-nitrophenyl)-4-pyrimidinyl]-p-toluenesulphonamide,
p-(benzyloxy)-N-[5-(p-chlorophenyl)-6-(2-hydroxyethoxy)-4-pyrimidinyl]benzenesulphonamide,
N-[5-(p-chlorophenyl)-4-pyrimidinyl]-p-hydroxybenzenesulphonamide,
N-[5-(p-chlorophenyl)-6-(2-hydroxyethoxy)-4-pyrimidinyl]-p-(2-methoxyethoxy)benzenesulphonamide,
N-[5-(p-bromophenyl)-6-(2-hydroxyethoxy)-4-pyrimidinyl]-p-chlorobenzenesulphonamide,
p-chloro-N-[6-(2-hydroxyethoxy)-5-p-tolyl-4-pyrimidinyl]benzenesulphonamide,
N-[5-(p-chlorophenyl)-6-(2-hydroxyethoxy)-4-pyrimidinyl]-α,α,α-trifluoro-p-toluenesulphonamide sodium salt,
N-[6-(2-hydroxyethoxy)-5-(p-methoxyphenyl)-4-pyrimidinyl]-p-toluenesulphonamide,
N-[6-(2-hydroxyethoxy)-5-(p-methoxyphenyl)-4-pyrimidinyl]-p-methoxybenzenesulphonamide,
N-[6-(2-hydroxyethoxy)-5-(p-methoxyphenyl)-4-pyrimidinyl]-p-(methylthio)benzenesulphonamide,
N-[6-(2-hydroxyethoxy)-5-(p-methoxyphenyl)-2-methyl-4-pyrimidinyl]-p-methoxybenzenesulphonamide,
N-[6-(2-hydroxyethoxy)-5-(p-methoxyphenyl)-4-pyrimidinyl]-p-isopropylbenzenesulphonamide,
p-t-butyl-N-[6-(2-hydroxyethoxy)-5-(p-methoxyphenyl)-4-pyrimidinyl]benzenesulphonamide,
rac-p-sec-butyl-N-[6-(2-hydroxyethoxy)-5-(p-methoxyphenyl)-4-pyrimidinyl]benzenesulphonamide,
N-[6-(2-hydroxyethoxy)-5-[p-(methylthio)phenyl]-4-pyrimidinyl]-p-isopropylbenzenesulphonamide,
N-[6-(2-hydroxyethoxy)-5-[p-(methylthio)phenyl]-4-pyrimidinyl]-α,α,α-trifluoro-p-toluenesulphonamide,
p-chloro-N-[5-(p-chlorophenyl)-6-(2-hydroxyethoxy)-2-methyl-4-pyrimidinyl]benzenesulphonamide,
p-chloro-N-[6-(2-hydroxyethoxy)-5-(p-nitrophenyl)-4-pyrimidinyl]benzenesulphonamide,
N-[5-(p-aminophenyl)-6-(2-hydroxyethoxy)-4-pyrimidinyl]-p-chlorobenzenesulphonamide hydrochloride,
N-[5-(4-biphenylyl)-6-(2-hydroxyethoxy)-4-pyrimidinyl]-p-chlorobenzenesulphonamide,
p-chloro-N-[(6-hydroxyethoxy)-5-(α,α,α-trifluoro-p-tolyl)-4-pyrimidinyl]benzenesulphonamide,
p-chloro-N-[5-(p-hydroxyphenyl)-6-(2-hydroxyethoxy)-4-pyrimidinyl]benzenesulphonamide,
N-[5-[p-(benzyloxy)phenyl]-6-(2-hydroxyethoxy)-4-pyrimidinyl]-p-chlorobenzenesulphonamide,
N-[6-(2-hydroxyethoxy)-5-(α,α,α-trifluoro-p-tolyl)-4-pyrimidinyl]-α,α,α-trifluoro-p-toluenesulphonamide,
N-[5-(p-chlorophenyl)-6-(2-hydroxyethoxy)-4-pyrimidinyl]-p-isopropylbenzenesulphonamide,
N-[6-hydroxyethoxy)-5-p-tolyl-4-pyrimidinyl]-p-isopropylbenzenesulphonamide,
p-tert-butyl-N-[6-(2-hydroxyethoxy)-5-p-tolyl-4-pyrimidinyl]benzenesulphonamide,
N-[6-(2-hydroxyethoxy)-5-p-tolyl-4-pyrimidinyl]-p-(2-methoxyethoxy)benzenesulphonamide,
N-[6-(2-hydroxyethoxy)-5-p-tolyl-4-pyrimidinyl]-p-(trifluoromethoxy)benzenesulphonamide,
p-butyl-N-[6-(2-hydroxyethoxy)-5-p-tolyl-4-pyrimidinyl]-benzenesulphonamide,
N-[6-(2-hydroxyethoxy)-5-p-tolyl-4-pyrimidinyl]-2-naphthalenesulphonamide,
N-[6-(2-hydroxyethoxy)-5-p-tolyl-4-pyrimidinyl]-p-toluenesulphonamide,
N-[6-(2-hydroxyethoxy)-5-p-tolyl-4-pyrimidinyl]-α,α,α-trifluoro-p-toluenesulphonamide,
p-(2-hydroxyethoxy)-N-[6-(2-hydroxyethoxy)-5-p-tolyl-4-pyrimidinyl]benzenesulphonamide,
N-[6-(2-hydroxyethoxy)-5-p-tolyl-4-pyrimidinyl]-p-propylbenzenesulphonamide,
N-[6-(2-hydroxyethoxy)-5-p-tolyl-4-pyrimidinyl]-o-propylbenzenesulphonamide,
p-ethyl-N-[6-(2-hydroxyethoxy)-5-p-tolyl-4-pyrimidinyl]benzenesulphonamide,
o-ethyl-N-[6-(2-hydroxyethoxy)-5-p-tolyl-4-pyrimidinyl]benzenesulphonamide,
p-cyclopentyl-N-[6-(2-hydroxyethoxy)-5-p-tolyl]benzenesulphonamide,
α,α,α-trifluoro-N-[6-(2-hydroxyethoxy)-5-p-tolyl-4-pyrimidinyl]-o-toluenesulphonamide,
N-[6-(2-hydroxyethoxy)-5-p-tolyl-4-pyrimidinyl]-o-toluenesulphonamide,
p-chloro-N-[6-(2-hydroxyethoxy)-5-(1-naphthylmethyl)-4-pyrimidinyl]benzenesulphonamide,
N-6-(2-hydroxyethoxy)-5-(p-isopropylphenyl)-4-pyrimidinyl]-p-isopropylbenzenesulphonamide,
p-cyclopentyl-N-[6-(2-hydroxyethoxy)-5-(p-isopropylphenyl)-4-pyrimidinyl]benzenesulphonamide,
N-[6-(2-hydroxyethoxy)-5-(α,α,α-trifluoro-p-tolyl)-4-pyrimidinyl]-p-isopropylbenzenesulphonamide,
N-[5-(p-bromophenyl)-6-(2-hydroxyethoxy)-4-pyrimidinyl]-p-isopropylbenzenesulphonamide,
N-[6-(2-hydroxyethoxy)-5-(p-ethylphenyl)-4-pyrimidinyl]-p-isopropyl]benzenesulphonamide,
p-cyclopentyl-N-[6-(2-hydroxyethoxy)-5-(p-ethylphenyl)-4-pyrimidinyl]benzenesulphonamide.

## Revendications

1. Application de composés de formule 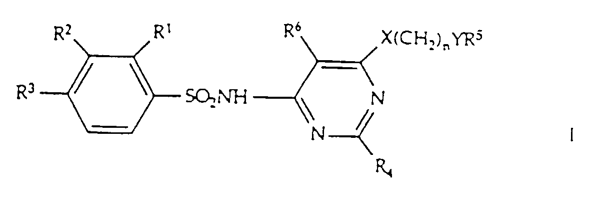 dans laquelle
R¹ représente un hydrogène, un alkyle inférieur, un alcoxy inférieur, un alkyle inférieur-thio, un halogène ou un trifluorométhyle;
R² représente un hydrogène, un halogène, un alcoxy inférieur, un hydroxy-alcoxy inférieur ou un trifluorométhyle;
R³ représente un hydrogène, un hydroxy, un halogène, un alkylthio, un cycloalkyle, un hydroxy-alkyle inférieur, un hydroxy-alcoxy inférieur, un hydroximino-alkyle inférieur, un alcényle inférieur, un oxo-alkyle inférieur, un trifluorométhyle, un trifluorométhoxy, un alcoxy inférieur, un alcoxy inférieur-alcoxy inférieur ou un aryl-alcoxy inférieur;
R² et R³ représentent ensembble un butadiényle;
R⁴ représente un hydrogène, un alkyle inférieur, un aryle ou un hétéroaryle;
R⁵ représente un hydrogène, un alcanoyle inférieur, un benzoyle, un hétérocyclyl-carbonyle ou un tétrahydropyran-2-yle;
R⁶ représente un radical de formule 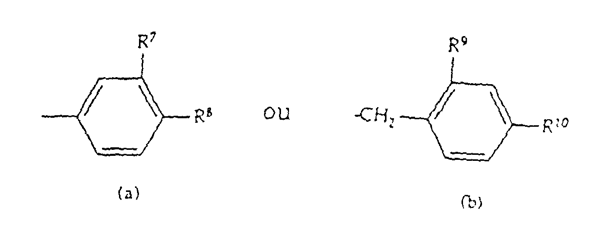
R⁷ représente un hydrogène, un alcoxy inférieur ou un nitro;
R⁸ représente un hydrogène, un halogène, un alkyle inférieur, un alcoxy inférieur, un alkyle inférieur thio, un nitro, un hydroxy, un amino ou un trifluorométhyle;
R⁷ et R⁸ représentent ensemble un butadiényle;
R⁹ représente un hydrogène, un halogène, un alkyle inférieur, un alcoxy inférieur, un alkyle inférieur thio, ou un trifluorométhyle;
R¹⁰ représente un hydrogène, un halogène, un alkyle inférieur, un alcoxy inférieur ou un alkyle inférieur-thio;
X et Y représentent indépendamment l'un de l'autre O, S ou NH; et
n vaut 2, 3 ou 4;
où les radicaux "inférieurs" désignent des radicaux en C₁ à C₇;
et de leurs sels comme substances actives dans la préparation de médicaments pour le traitement des troubles circulatoires, en particulier l'hypertonie, l'ischémie, les angiospasmes et l'angine de poitrine.

2. Application selon la revendication 1 de composés de formule I, et de leurs sels, dans laquelle R⁴ est un hydrogène, un alkyle inférieur ou un aryle et les autres symboles ont la signification donnée dans la revendication 1.

3. Application selon la revendication 1 de composés de formule I de la revendication 1 et de leurs sels, dans laquelle R⁶ représente un radical de formule 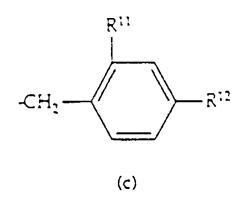 et R¹¹ représente un halogène, un alcoxy inférieur, un alkyle inférieur-thio ou un trifluorométhyle; et R¹² représente un hydrogène ou un alcoxy inférieur et R¹-R⁵, X, Y et n ont la signification indiquée dans la revendication 1.

4. Application selon la revendication 1 de composés de formule I et de leurs sels, dans laquelle R⁴ représente un hydrogène, un alkyle inférieur ou un aryle et les autres symboles ont la signification indiquée dans la revendication 3.

5. Application selon la revendication 1 des composés
N-[5-(2,6-diméthoxybenzyl)-6-(3-hydroxypropyl)-4-pyrimidinyl]-p-vinylbenzènesulfonamide,
α,α,α-trifluoro-N-[6-(2-hydroxyéthoxy)-6-[o-(trifluorométhyl)benzyl]pyrimidinyl]-p-toluènesulfonamide,
N-[6-(2-hydroxyéthoxy)-5-[o-(trifluorométhyl)benzyl]-4-pyrimidinyl]-p-méthoxybenzènesulfonamide,
p-chloro-N-[6-(2-hydroxyéthoxy)-5-[o-(trifluorométhyl)benzyl]-4-pyrimidinyl]benzènesulfonamide,
N-[6-(2-hydroxyéthoxy)-5-(o-méthoxybenzyl)-4-pyrimidinyl]-p-vinylbenzènesulfonamide,
N-[6-(2-hydroxyéthoxy)-5-[o-(trifluorométhyl)benzyl]-4-pyrimidinyl]-p-(méthylthio)benzènesulfonamide,
N-[5-(2,4-diméthoxybenzyl)-6-(2-hydroxyéthoxy)-4-pyrimidinyl]-p-isopropylbenzènesulfonamide,
rac-N-[5-(o-méthoxybenzyl)-6-[2-[(tétrahydro-2H-pyran-2-yl)oxy] éthoxy]-4-pyrimidinyl]-p-vinylbenzènesulfonamide,
rac-p-[[5-(o-méthoxybenzyl)-6-[2-[(tétrahydro-2H-pyran-2-yl)oxy]éthoxy]-4-pyrimidinyl]sulfamoyl]benzaldéhyde,
p-[(RS)-1-hydroxyéthyl]-N-[5-(-méthoxybenzyl)-6-[2-[[(RS)-tétrahydro-2H-pyran-2-yl]oxy]éthoxy]-4-pyrimidinyl]benzènesulfonamide,
rac-α-hydroxy-N-[5-(o-méthoxybenzyl)-6-[2-[(tétrahydro-2H-pyran-2-yl)oxy]éthoxy]-4-pyrimidinyl]-p-toluènesulfonamide,
rac-α-[(>E/Z)-hydroxyimino-N-[5-(o-méthoxybenzyl)-6-[2-[(tétrahydro-2H-pyran-2-yl)oxy]éthoxy]-4-pyrimidinyl]-p-toluènesulfonamide,
rac-N-6-(2-hydroxyéthoxy)-5-(o-méthoxybenzyl)-p-(1-hydroxyéthyl)-benzènesulfonamide,
α-[(E/Z)-hydroxyimino]-N-[6-(2-hydroxyéthoxy)-5-(o-méthoxybenzyl)-4-pyrimidinyl]-p-toluènesulfonamide,
p-[[6-(2-hydroxyéthoxy)-5-(o-méthoxybenzyl)-4-pyrimidinyl]sulfamoyl]-benzaldéhyde,
2-[[5-(o-méthoxybenzyl)-6-[(p-vinylphényl)-sulfamoyl]-4-pyrimidinyl]-oxy]éthylacétate,
N-[6-(2-hydroxyéthoxy)-5-(o-méthoxybenzyl)-4-pyrimidinyl]-p-méthoxybenzènesulfonamide,
N-[6-(2-hydroxyéthoxy)-5-(o-chlorobenzyl)-4-pyrimidinyl]-p-toluènesulfonamide,
N-[6-(2-hydroxyéthoxy)-5-(o-méthoxybenzyl)-4-pyrimidinyl]-p-méthylthiobenzènesulfonamide,
N-[6-(2-hydroxyéthoxy)-5-(o-méthoxybenzyl)-4-pyrimidinyl]-α,α,α-trifluoro-p-toluènesulfonamide,
N-[6-(2-hydroxyéthoxy)-5-(o-méthoxybenzyl)-4-pyrimidinyl]-p-isopropylbenzènesulfonamide,
p-t-butyl-N-[6-(2-hydroxyéthoxy)-5-(o-méthoxybenzyl)-4-pyrimidinyl]-benzènesulfonamide,
N-[6-(2-hydroxyéthoxy)-5-(o-chlorobenzyl)-4-pyrimidinyl]-p-isopropylbenzènesulfonamide,
N-[6-(2-hydroxyéthoxy)-5-(o-méthylthiobenzyl)-4-pyrimidinyl]-p-isopropylbenzènesulfonamide,
N-[6-(2-hydroxyéthoxy)-5-(o-chlorobenzyl)-4-pyrimidinyl]-p-isobutylbenzènesulfonamide,
N-[6-(2-hydroxyéthoxy)-5-(o-chlorobenzyl)-4-pyrimidinyl]-p-cyclohexylbenzènesulfonamide,
N-[6-(2-hydroxyéthoxy)-5-(o-chlorobenzyl)-4-pyrimidinyl]-p-isopentylbenzènesulfonamide,
N-[6-(2-hydroxyethoxy)-5-(o-méthoxybenzyl)-4-pyrimidinyl]-p-isopropylthiobenzènesulfonamide.

6. Application selon la revendication 1 de composés de formule I de la revendication 1 et de leurs sels, dans laquelle R⁶ représente un radical de formule 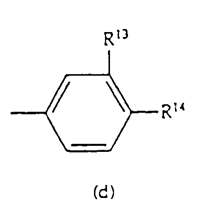 et R¹³ représente un hydrogène, un alcoxy inférieur ou un nitro, et R¹⁴ représente un hydrogène, un halogène, un alkyle inférieur; un alcoxy inférieur, alkyle inférieur-thio ou un nitro, ou R¹³ et R¹⁴ représentent ensemble un butadiényle, et R¹-R⁵ , X, Y et n ont la signification donnée dans la revendication 1.

7. Application selon la revendication 1 de composés de formule I et de leurs sels, dans laquelle R⁴ représente un hydrogène, un alkyle inférieur ou un aryle et les autres symboles ont la signification donnée dans la revendication 6.

8. Application selon la revendication 1 des composés:
N-[5-(p-chlorophényl)-6-(2-hydroxyéthoxy)-4-pyrimidinyl]-α,α,α-trifluoro-p-toluènesulfonamide,
N-[5-(p-chlorophényl)-6-(2-hydroxyéthoxy)-4-pyrimidinyl]-p-(trifluorométhoxy)-benzènesulfonamide,
p-chloro-N-[5-(m-chlorophényl)-6-(2-hydroxyéthoxy)-4-pyrimidinyl]benzènesulfonamide,
p-chloro-N-[5-(p-fluorophényl)-6-(2-hydroxyéthoxy)-4-pyrimidinyl]benzènesulfonamide,
N-[5-(p-chlorophényl)-6-(2-hydroxyéthoxy)-4-pyrimidinyl]-p-fluorobenzènesulfonamide,
o-chloro-N-[5-(p-chlorophényl)-6-(2-hydroxyéthoxy)-4-pyrimidinyl]benzènesulfonamide,
p-chloro-N-[5-(3,4-diméthoxyphényl)-6-(2-hydroxyéthoxy)-4-pyrimidinyl]benzènesulfonamide,
3,4-dichloro-N-[5-(p-chlorophényl)-6-(2-hydroxyéthoxy)-4-pyrimidinyl]benzènesulfonamide,
N-[5-(p-chlorophényl)-6-(2-hydroxyéthoxy)-4-pyrimidinyl]-α,α,α-α',α',α'-hexafluoro-3,5-xylènesulfonamide,
3-chloro-N-[5-(p-chlorophényl)-6-(2-hydroxyéthoxy)-4-pyrimidinyl]-4(2-hydroxyéthoxy)benzènesulfonamide,
p-chloro-N-[6-(2-hydroxyéthoxy)-5-(p-nitrophényl)-4-pyrimidinyl]benzènesulfonamide,
p-butoxy-N-[5-(p-chlorophényl)-6-(2-hydroxyéthoxy)-4-pyrimidinyl]benzènesulfonamide,
N-[5-(p-chlorophényl)-6-(2-hydroxyéthoxy)-4-pyrimidinyl]-3,4-diméthoxy-benzènesulfonamide,
2-chloro-N-[5-(p-chlorophényl)-6-(2-hydroxyéthoxy)-4-pyrimidinyl]-α,α,α-trifluoro-p-toluènesulfonamide,
6-chloro-N-[5-(p-chlorophényl)-6-(2-hydroxyéthoxy)-4-pyrimidinyl]-α,α,α-trifluoro-m-toluènesulfonamide,
2,3,4-trichloro-N-[(5-chlorophényl)-6-(2-hydroxyéthoxy)-4-pyrimidinyl]benzènesulfonamide,
m-chloro-N-(p-chlorophényl)-6-(2-hydroxyéthoxy)-4-pyrimidinyl]-benzènesulfonamide,
2,4-dichloro-N-[5-(p-chlorophényl)-6-(2-hydroxyéthoxy)-4-pyrimidinyl]-benzènesulfonamide,
N-5-(p-chlorophényl)-6-(2-hydroxyéthoxy)-α,α,α-trifluoro-m-toluènesulfonamide,
N-[5-(p-chlorophényl)-6-(2-hydroxyéthoxy)-4-pyrimidinyl]-α,α,α-trifluoro-o-toluènesulfonamide,
N-[5-(p-chlorophényl-)-6-(2-hydroxyéthoxy)-4-pyrimidinyl]-2-naphtalènesulfonamide,
p-chloro-N-[6-(2-hydroxyéthoxy)-5-(m-nitrophényl)-4-pyrimidinyl]benzènesulfonamide,
α,α,α-trifluoro-N-[6-(2-hydroxyéthoxy)-5-(m-nitrophényl)-4-pyrimidinyl]-p-toluènesulonamide,
p-(benzyloxy)-N-[5-(chlorophényl)-6-(2-hydroxyéthoxy)-4-pyrimidinyl]-benzènesulfonamide,
N-[5-(p-chlorophényl)-4-pyrimidinyl]-p-hydroxybenzènesulfonamide,
N-[5-(p-chlorophényl)-6-(2-hydroxyéthoxy)-4-pyrimidinyl]-p-(2-méthoxyéthoxy)benzènesulfonamide,
N-[5-(p-bromophényl)-6-(2-hydroxyéthoxy)-4-pyrimidinyl]-p-chlorobenzènesulfonamide,
p-chloro-N-[6-(2-hydroxyéthoxy)5-p-tolyl-4-pyrimidinyl]benzènesulfonamide,
N-[5-(p-chlorophényl)-6-(2-hydroxyéthoxy)-4-pyrimidinyl]-α,α,α-trifluoro-p-toluènesulfonamide, sel de sodium
N-[6-(2-hydroxyéthoxy)-5-(p-méthoxyphényl)-4-pyrimidinyl]-p-toluènesulfonamide,
N-[6-(2-hydroxyéthoxy)-5-(p-méthoxyphényl)-4-pyrimidinyl]-p-méthoxybenzènesulfonamide,
N-[6-(2-hydroxyéthoxy)-5-(p-méthoxyphényl)-4-pyrimidinyl]-p-méthylthio)benzènesulfonamide,
N-[6-(2-hydroxyéthoxy)-5-(p-méthoxyphényl)-2-méthyl-4-pyrimidinyl]-p-méthoxybenzènesulfonamide,
N-[6-(2-hydroxyéthoxy)-5-(p-méthoxyphényl)-4-pyrimidinyl]-p-isopropyl-benzènesulfonamide,
p-t-butyl-N-[6-(2-hydroxyéthoxy)-5-(p-méthoxyphényl)-4-pyrimidinyl]benzènesulfonamide,
rac-p-sec-butyl-N-[6-(2-hydroxyéthoxy)-5-(p-méthoxyphényl)-4-pyrimidinyl]benzènesulfonamide,
N-[6-(2-hydroxyéthoxy)-5-[p-(méthylthio)phényl]-4-pyrimidinyl]-p-isopropylbenzènesulfonamide,
N-[6-(2-hydroxyéthoxy)-5-[p-(méthylthio)phényl]-4-pyrimidinyl]-α,α,α-trifluoro-p-toluènesulfonamide,
p-chloro-N-[5-(p-chlorophényl)-6-(2-hydroxyéthoxy)-2-méthyl-4-pyrimidinyl]benzènesulfonamide,
p-chloro-N-[6-(2-hydroxyéthoxy)-5(p-nitrophényl)-4-pyrimidinyl]benzènesulfonamide,
N-[5-(p-aminophényl)-6-(2-hydroxyéthoxy)-4-pyrimidinyl]-p-chlorobenzènesulfonamide, chlorhydrate
N-[5-(4-biphénylyl)-6-(2-hydroxyéthoxy)-4-pyrimidinyl]-p-chlorobenzènesulfonamide,
p-chloro-N-[(6-hydroxyéthoxy)-5-(α,α,α-trifluoro-p-tolyl)-4-pyrimidinyl]-benzènesulfonamide,
p-chloro-N-[5-(p-hydroxyphényl)-6-(2-hydroxyéthoxy)-4-pyrimidinyl]-benzènesulfonamide,
N-[5-(p-benzyloxy)phényl]-6-(2-hydroxyéthoxy)-4-pyrimidinyl]-p-chlorobenzènesulfonamide,
N-[6-(2-hydroxyéthoxy)-5-(α,α,α-trifluoro-p-tolyl)-4-pyrimidinyl]-α,α,α-trifluoro-p-toluènesulfonamide,
N-[5-(p-chlorophényl)-6-(2-hydroxyéthoxy)-4-pyrimidinyl]-p-isopropyl-benzènesulfonamide,
N-[(6-hydroxyéthoxy)-5-p-tolyl-4-pyrimidinyl]-p-isopropylbenzènesulfonamide,
p-tert-butyl-N-[6-(2-hydroxyéthoxy)-5-p-tolyl-4-pyrimidinyl]benzènesulfonamide,
N-[6-(2-hydroxyéthoxy)-5-p-tolyl-4-pyrimidinyl]-p-(2-méthoxyéthoxy)-benzènesulfonamide,
N-[6-(2-hydroxyéthoxy)-5-p-tolyl-4-pyrimidinyl]-p-(2-trifluorométhoxy)-benzènesulfonamide,
p-butyl-N-[6-(2-hydroxyéthoxy)-5-p-tolyl-4-pyrimidinyl]benzènesulfonamide,
N-[6-(2-hydroxyéthoxy)-5-p-tolyl-4-pyrimidinyl]-2-naphtalènesulfonamide,
N-[6-(2-hydroxyéthoxy)-5-p-tolyl-4-pyrimidinyl]-p-toluènesulfonamide,
N-[6-(2-hydroxyéthoxy)-5-p-tolyl-4-pyrimidinyl]-α,α,α-trifluoro-p-toluènesulfonamide,
p-(2-hydroxyéthoxy)-N-[6-(2-hydroxyéthoxy)-5-p-tolyl-4-pyrimidinyl]-benzènesulfonamide,
N-[6-(2-hydroxyéthoxy)-5-p-tolyl-4-pyrimidinyl]-p-propylbenzènesulfonamide,
N-[6-(2-hydroxyéthoxy)-5-p-tolyl-4-pyrimidinyl]-o-propylbenzènesulfonamide,
p-éthyl-N-[6-(2-hydroxyéthoxy)-5-p-tolyl-4-pyrimidinyl]benzènesulfonamide,
o-éthyl-N-[6-(2-hydroxyéthoxy)-5-p-tolyl-4-pyrimidinyl]benzènesulfonamide,
p-cyclopentyl-N-[6-(2-hydroxyéthoxy)-5-p-tolyl]benzènesulfonamide,
α,α,α-trifluoro-N-[6-(2-hydroxyéthoxy)-5-p-tolyl-4-pyrimidinyl]-o-toluènesulfonamide,
N-[6-(2-hydroxyéthoxy)-5-p-tolyl-4-pyrimidinyl]-o-toluènesulfonamide,
p-chloro-N-[6-(2-hydroxyéthoxy)-5-(1-naphtylméthyl)-4-pyrimidinyl]-benzènesulfonamide,
N-6-(2-hydroxyéthoxy)-5-(p-isopropylphényl)-4-pyrimidinyl]-p-isopropylbenzènesulfonamide,
p-cyclopentyl-N-[6-(2-hydroxyéthoxy)-5-(p-isopropylphényl)-4-pyrimidinyl]benzènesulfonamide,
N-6-(2-hydroxyéthoxy)-5-(α,α,α-trifluoro-p-tolyl)-4-pyrimidinyl]-p-isopropylbenzènesulfonamide,
N[5-(p-bromophényl)-6-(2-hydroxyéthoxy)-4-pyrimidinyl]-p-isopropylbenzènesulfonamide,
N-[6-(2-hydroxyéthoxy)-5-(p-éthylphényl)-4-pyrimidinyl]-p-isopropyl]-benzènesulfonamide,
p-cyclopentyl-N-[6-(2-hydroxyéthoxy)-5-(p-éthylphényl)-4-pyrimidinyl]-benzènesulfonamide.
